(19) Europäisches Patentamt
European Patent Office
Office européen des brevets

(11) Veröffentlichungsnummer : **0 073 876**
**B1**

# (12) EUROPÄISCHE PATENTSCHRIFT

(45) Veröffentlichungstag der Patentschrift : **11.12.85**

(51) Int. Cl.⁴ : **D 06 P 5/12**, D 06 P 5/17, C 09 B 29/01// D06P1/18

(21) Anmeldenummer : **82102395.9**

(22) Anmeldetag : **23.03.82**

## (54) Verfahren zur Herstellung von Reserveeffekten auf Polyester- und Polyester-Zellulose-Mischfaser-Textilien.

(30) Priorität : **08.09.81 DE 3135433**

(43) Veröffentlichungstag der Anmeldung : **16.03.83 Patentblatt 83/11**

(45) Bekanntmachung des Hinweises auf die Patenterteilung : **11.12.85 Patentblatt 85/50**

(84) Benannte Vertragsstaaten : **BE CH DE FR GB IT LI**

(56) Entgegenhaltungen :
**EP-A- 0 038 615**
**EP-A- 0 041 697**
**DE-A- 2 856 283**
**GB-A- 1 543 724**
**RESEARCH DISLCOSURE, Band 198, Oktober 1980, Seiten 415-416, Nr. 19827, Havant, Hamsphire (GB); "A process for the discharge printing of aromatic polyester textile materials"**

(73) Patentinhaber : **CASSELLA Aktiengesellschaft**
**Hanauer Landstrasse 526**
**D-6000 Frankfurt am Main 61 (DE)**

(72) Erfinder : **Bühler, Ulrich, Dr.**
**Nidderauer Strasse 13**
**D-6369 Schöneck 1 (DE)**
Erfinder : **Kindler, Horst, Dr.**
**Birsteinerstrasse 14**
**D-6000 Frankfurt/Main 61 (DE)**
Erfinder : **Kühlein, Klaus, Dr.**
**Fasanenstrasse 41**
**D-6233 Kelkheim/Ts (DE)**
Erfinder : **Kallay, Maria, Dipl.-Ing.**
**Kastanienweg 7d**
**D-6240 Königstein 3 (DE)**
Erfinder : **Roth, Kurt**
**Breckenheimer Strasse 35**
**D-6238 Hofheim (DE)**
Erfinder : **Kosubek, Uwe**
**Am Vogelanger 28**
**D-6087 Büttelborn (DE)**
Erfinder : **Löwenfeld, Rudolf, Dr.**
**Quellenweg 2**
**D-6072 Dreieich (DE)**

(74) Vertreter : **Urbach, Hans-Georg, Dr. et al**
**Hanauer Landstrasse 526**
**D-6000 Frankfurt am Main 61 (DE)**

**EP 0 073 876 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Auf dem Gebiet des Textildrucks war es seit jeher ein Problem, weiße oder farbige scharf begrenzte Muster auf tiefgefärbtem Hintergrund zu erzeugen. Insbesondere, wenn die Herstellung filigranartiger Muster auf dunklem Untergrund gewünscht wird, versagt der direkte Druck des Textilmaterials völlig. Um solche Dessins herzustellen, ist es seit langem bekannt, auf einer mit einem weißätzbaren Farbstoff hergestellten tiefen Hintergrundfärbung eine Ätzpaste in dem gewünschten Muster aufzudrucken und anschließend durch eine trockene oder nasse Wärmebehandlung den Farbstoff an den mit der Ätzpaste bedruckten Stellen zu zerstören. Nach dem Auswaschen der so erhaltenen Drucke wird das gewünschte Muster weiß auf dunklem Fond erhalten. Es ist auch bereits bekannt, den Ätzdruckpasten Farbstoffe zuzusetzen, die gegen das Ätzmittel resistent sind. In diesem Fall wird gleichzeitig mit der Zerstörung der Fondfärbung eine Färbung des Textilmaterials an den bedruckten Stellen durch den unzerstörbaren Farbstoff vorgenommen. Man erhält in diesem Fall farbige Drucke auf dunklem Fond. Farbige Drucke auf dunklem Fond können auch erhalten werden, wenn der dunkle Fond mit einer Mischung eines ätzbaren und eines andersfarbigen nichtätzbaren Farbstoffs hergestellt wird, indem man beide Farbstofftypen in die Klotzflotte einbringt.

Aus der Deutschen Offenlegungschrift 2 836 391 ist ein Verfahren bekannt zur Herstellung von Ätzreservedrucken auf Textilmaterialien, insbesondere solchen, die hydrophobe Fasern, vorzugsweise Polyesterfasern, in überwiegendem Maß, enthalten oder aus solchen Fasern bestehen, durch Imprägnieren der Materialien mit Farbflotten, die neben üblichen Färbe- und Klotzhilfsmitteln weißätzbare Farbstoffe und gegebenenfalls ätzmittelbeständige Farbstoffe enthalten, Trocknen oder Antrocknen der geklotzten Materialien und anschließendes Aufdrucken einer Ätzreservepaste, die gewünschtenfalls neben dem Ätzmittel noch ätzmittelbeständige Farbstoffe enthält und anschließende Wärmebehandlung bei Temperaturen von 100 bis 230 °C. Als weißätzbare Farbstoffe werden solche der Formel

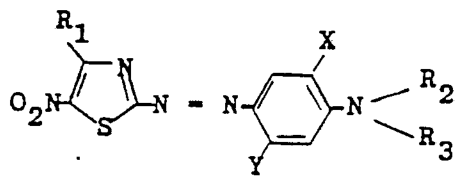

worin $R_1$, $R_2$, X und Y Wasserstoff oder einen einwertigen Substituenten und $R_3$ einen einwertigen Kohlenwasserstoffrest bedeuten, verwendet. Als Ätzmittel werden Basen eingesetzt, die in 5 %iger wäßriger Lösung mindestens einen pH-Wert von 8 hervorbringen, wie zum Beispiel Hydroxide der Alkali- und Erdalkalimetalle, Salze von Erdalkali- und Alkalimetallen mit schwachen organischen oder anorganischen Säuren, Ammoniak oder auch aliphatische Amine. Vorzugsweise wird als Base in den Ätzreservedruckpasten Natrium- oder Kaliumhydroxyd oder Natrium- oder Kaliumcarbonat oder Natrium- oder Kaliumbicarbonat verwendet.

Aus der Deutschen Offenlegungsschrift Nr. 2 856 283 ist es bekannt, Reserveeffekte auf Textilmaterialien auf Basis von Mischfasern aus Polyester und Cellulose, insbesondere Polyester-Baumwollfasern, herzustellen durch Imprägnieren der Materialien mit Farbflotten, die neben üblichen Färbe- und Klotzhilfsmitteln Dispersions- und Reaktivfarbstoffe enthalten, die mit den Reservierungsmitteln reagieren und die gegebenenfalls weitere gegen die Reservierungsmittel beständige Dispersions- und Reaktivfarbstoffe enthalten, Trocknen oder Antrocknen der geklotzten Materialien und anschließendes Aufdrucken einer Reservepaste, die gewünschtenfalls neben dem Reservierungsmittel gegen das Reservierungsmittel beständige Dispersions- und Reaktivfarbstoffe enthält, Wärmebehandlung bei Temperaturen von 100 bis 190 °C und daran anschließendes alkalisches Fixieren des Reaktivfarbstoffs in an sich bekannter Weise. Bei diesem bekannten Verfahren werden als ätzbare Dispersionsfarbstoffe ebenfalls solche der Formel

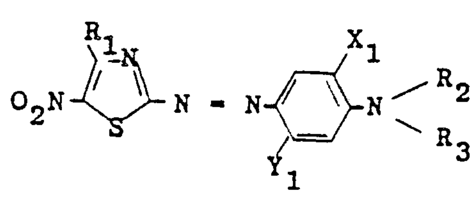

worin $R_1$, $R_2$, $X_1$ und $Y_1$ Wasserstoff oder einen einwertigen Substituent und $R_3$ einen einwertigen Kohlenwasserstoffrest bedeuten, oder solche der Formel

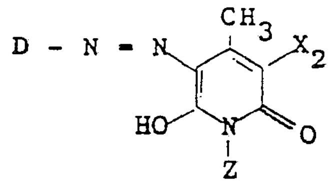

worin D für Phenyl steht, das gegebenenfalls mindestens einen Substituenten enthält ; $X_2$ Wasserstoff oder einen einwertigen Substituent und Z Wasserstoff oder einen einwertigen Kohlenwasserstoffrest bedeuten, eingesetzt. Als ätzbare Reaktivfarbstoffe verwendet man hierbei solche, die reaktive Reste der folgenden Formeln :

$—SO_2—CH_2—CH_2—hal$
$—SO_2—CH_2—CH_2—O—SO_3M$
$—NH—SO_2—CH_2—CH_2—O—SO_3M$
$—SO_2—CH=CH_2$

worin M Wasserstoff oder ein Metallkation und hal Halogen bedeuten, enthalten. Die bei diesem Verfahren eingesetzte Reservepaste enthält als Reservierungsmittel a) ein Alkalisulfit oder aber ein Alkalihydrogensulfit in Kombination mit Alkalicarbonat oder Alkalihydrogencarbonat und gegebenenfalls einem Aldehyd und b) gegebenenfalls ein nichtionogenes Detergens.

Aus der Deutschen Offenlegungsschrift 3 019 726 ist es bekannt, daß man nach dem Verfahren der DE-A-2 856 283 auch sehr gute Reserveeffekte auf den genannten Textilmaterialien erhält, wenn man als ätzbare Dispersionsfarbstoffe solche der Formel

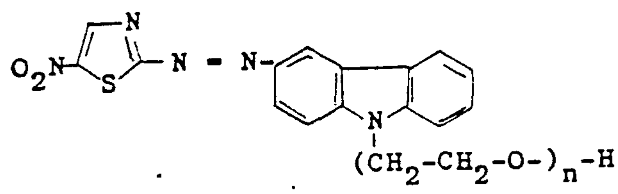

worin n eine Zahl von 1 bis 9, vorzugsweise 1 bis 5, bedeutet, einsetzt.

Den UK-Patentanmeldungen 2 071 707 und 2 071 708 ist zu entnehmen, daß bei der Durchführung der bekannten Ätzreservedruck-Verfahren « naß in naß » — z. B. wenn bei den Verfahren der DE-A-2 836 391 oder 2 856 283 nur kurz oder gar nicht angetrocknet wird — zweckmäßigerweise als alkalisch reagierende Substanz Natrium- oder Kaliumsilikat eingesetzt wird.

Es wurde nun gefunden, daß man nach den alkalischen Ätzdruckverfahren der DE-A-28 36 391 und der DE-A-28 56 283 hervorragende Ergebnisse erhält, wenn als ätzbare Dispersionsfarbstoffe solche der Formel I

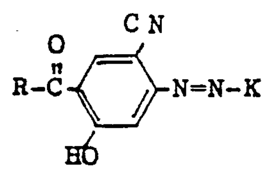

(I)

in der R und K die weiter unten genannten Bedeutungen haben, eingesetzt werden.

Die vorliegende Erfindung betrifft somit ein Verfahren zur Herstellung von weißen oder verschiedenfarbigen Mustern auf farbigem Untergrund auf Textilmaterialien, enthaltend oder bestehend aus hydrophoben synthetischen Fasern, insbesondere Polyesterfasern, oder auf Textilmaterialien auf Basis von Mischfasern aus Polyester und Cellulose, insbesondere Polyester-Baumwollfasern, durch Imprägnieren der Materialien mit Farbflotten, die neben den üblichen Färbe- und Klotzhilfsmitteln weißätzbare Dispersionsfarbstoffe, und, sofern Mischfasertextilien verarbeitet werden, gewünschtenfalls weißätzbare Reaktivfarbstoffe, die als Reaktivanker eine Gruppe der Formel

$—SO_2—CH_2—CH_2—hal$
$—SO_2—CH_2—CH_2—O—SO_3M$
$—NH—SO_2—CH_2—CH_2—O—SO_3M$
$—SO_2—CH=CH_2$

aufweisen, enthalten und Aufdrucken einer Ätzreservepaste, die gewünschtenfalls neben dem Ätzreservemittel noch ätzmittelbeständige Farbstoffe enthält, in dem gewünschten Muster, wobei die Reihenfolge

von Imprägnieren und Aufdrucken der Ätzreservepaste beliebig ist und das Imprägnieren auch durch Bedrucken mit einer entsprechenden Druckpaste ersetzt werden kann und anschließende Wärmebehandlung bei Temperaturen von 100 bis 230 °C, wobei für den Fall, daß keine weißätzbaren Reaktivfarbstoffe anwesend sind, als Ätzreservemittel eine Base, die in 5 %iger wäßriger Lösung mindestens einen pH-Wert von 8 hervorbringt oder ein Ätzreservemittel auf Sulfitbasis, das ein Alkalisulfit oder aber ein Alkalihydrogensulfit in Kombination mit Alkalicarbonat oder Alkalihydrogencarbonat und gegebenenfalls einem Aldehyd und gegebenenfalls ein nichtionogenes Detergens enthält, für den Fall, daß die obenbezeichneten weißätzbaren Reaktivfarbstoffe vorhanden sind, das Ätzreservemittel auf Sulfitbasis eingesetzt wird, dadurch gekennzeichnet, daß als ätzbare Dispersionsfarbstoffe solche der Formel I

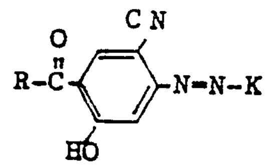

(I)

in der R gegebenenfalls substituiertes Alkyl mit 1 bis 8, vorzugsweise 1 bis 4 C-Atomen gegebenenfalls substituiertes Alkoxy mit 1 bis 8 C-Atomen, vorzugsweise 1 bis 4 C-Atomen und K den Rest einer Kupplungskomponente der Benzol-, Naphthalin-, Pyridin-, Benzpyridin-, Diazol-, Thiazol-, Indol-, Carbazol-, Oxazol-, Diazin-Reihe oder aus der Reihe der enolisierbaren 1,3-Dicarbonylverbindungen bedeuten und die in ihrem Molekül höchstens eine veresterte Carboxylgruppe aufweisen, eingesetzt werden.

Substituierte und unsubstituierte Alkyl- oder Alkoxyreste können, auch wenn sie als Substituenten auftreten, geradkettig oder verzweigt sein. Alkoxyreste, die für R stehen, sind zum Beispiel Methoxy, Ethoxy, n-Propoxy, i-Propoxy, n-Butoxy, i-Butoxy, sek. Butoxy, n-Pentoxy, i-Pentoxy, n-Hexoxy, i-Hexoxy, n-Heptoxy, i-Heptoxy, i-Octoxy.

Alkylreste, die für R stehen sind zum Beispiel : Methyl, Ethyl, n-Propyl, i-Propyl, n-Butyl, i-Butyl, sek.-Butyl, t-Butyl, n-Pentyl, i-Pentyl, n-Hexyl, i-Hexyl, n-Heptyl, i-Heptyl, i-Octyl.

Die Alkyl- und Alkoxyreste, die für R stehen, sind gegebenenfalls unabhängig voneinander ein oder zweifach substituiert durch Chlor, Brom, Cyan, Hydroxy, Alkoxy mit 1 bis 4 C-Atomen, Alkenoxy mit 3 oder 4 C-Atomen, Hydroxyalkoxy mit 2 bis 4-C-Atomen, Alkoxyalkoxy mit insgesamt 3 bis 8 C-Atomen, Alkylcarbonyloxy mit 2 bis 4 C-Atomen, gegebenenfalls substituiertes Benzoyloxy, gegebenenfalls substituiertes Phenyl, gegebenenfalls substituiertes Phenoxy. Als dritten Substituenten können die für R stehenden Alkyl- oder Alkoxygruppen eine OH-Gruppe tragen. Bevorzugt sind für R unsubstituierte oder monosubstituierte Alkyl- oder Alkoxygruppen.

Substituenten an gegebenenfalls substituierten Phenyl-, Phenoxy- oder Benzoylresten sind zum Beispiel : Alkyl mit 1 bis 4 C-Atomen, Alkoxy mit 1 bis 4 C-Atomen, Fluor, Chlor, Brom, Nitro, wobei Ein- und Zweifachsubstitution möglich ist ; bevorzugt sind jedoch die unsubstituierten Reste.

Besonders bevorzugt sind solche Bedeutungen von R, in denen mehrere bevorzugte Merkmale kombiniert sind.

Kupplungskomponenten KH der Benzol-Reihe sind Anilinderivate der Formel

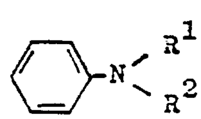

worin $R^1$ Wasserstoff, Alkyl, Alkenyl und $R^2$ Alkyl, Alkenyl, Cycloalkyl, Phenyl, Alkylcarbonyl, Benzoyl, Alkylsulfonyl, oder Phenylsulfonyl bedeutet, und deren N-Alkyl-, N-Alkenyl, N-Cycloalkyl, N-Phenyl-, N-Acyl-, N-Alkylsulfonyl- und N-Phenylsulfonylrest gegebenenfalls substituiert sein kann und deren Kern gegebenenfalls durch gegebenenfalls substituiertes Alkyl mit 1 bis 4, vorzugsweise 1 oder 2 C-Atomen, gegebenenfalls substituiertes Alkoxy mit 1 bis 4, vorzugsweise 1 oder 2 C-Atomen, Alkenoxy mit 3 oder 4 C-Atomen, gegebenenfalls substituiertes Phenoxy, gegebenenfalls substituiertes Phenyl, Fluor, Chlor, Brom, gegebenenfalls substituiertes Alkylcarbonylamino, gegebenenfalls substituiertes Benzoylamino, Alkenylcarbonylamino, gegebenenfalls substituiertes Alkylsulfonylamino, gegebenenfalls substituiertes Phenylsulfonylamino, gegebenenfals substituiertes N-Alkyl-N-alkyl-sulfonyl- oder N-Alkyl-N-phenylsulfonylamino, gegebenenfalls substituiertes Alkoxy-, Alkenoxy-, Cycloalkoxy- oder Phenoxycarbonylamino, gegebenenfalls substituiertes Alkyl-, Alkenyl-, Cycloalkyl- oder Phenylaminocarbonylamino oder Hydroxy ein- oder mehrfach substituiert sein kann, oder Phenole, deren Kern durch Alkyl mit 1 bis 4 C-Atomen oder gegebenenfalls substituiertes Alkylcarbonyl- oder Benzoylamino substituiert sein kann.

Kupplungskomponenten der Naphthalin-Reihe sind 1- oder 2-Naphthylamine der Formel

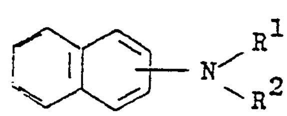

worin $R^1$ Wasserstoff, Alkyl, Alkenyl und $R^2$ Alkyl, Alkenyl, Cycloalkyl, Phenyl, Alkylcarbonyl, Benzoyl, Alkylsulfonyl oder Phenylsulfonyl bedeutet, wie z. B. N-mono-Alkyl-, N,N-di-Alkyl-, N-mono-Alkenyl-, N,N-di-Alkenyl-, N-Alkyl-N-alkenyl-, N-Cycloalkyl-, N-mono-Phenyl-, N-Phenyl-N-alkyl-, N-Phenyl-N-alkenyl-1- oder 2-Naphthylamine, deren N-Alkyl- oder N-Phenyl-Reste gegebenenfalls substituiert sein können und deren Kern gegebenenfalls substituiert sein kann durch Alkyl mit 1 bis 4 C-Atomen, Alkoxy mit 1 bis 4 C-Atomen, Chlor, Brom, Aminosulfonyl, N-mono- oder N,N-di-alkyl-substituiertes Aminosulfonyl, wobei die Alkylreste ihrerseits unabhängig voneinander gegebenenfalls ein- oder mehrfach substituiert sein können, oder 1- oder 2-Naphthole, deren Kern gegebenenfalls substituiert sein kann durch Aminocarbonyl, gegebenenfalls unabhängig voneinander substituiertes Mono- oder Dialkyl- oder Phenylaminocarbonyl, gegebenenfalls substituiertes Alkoxy-, Alkenoxy-, Cycloalkoxy- oder Phenoxycarbonyl, Amino, gegebenenfalls substituiertes Alkylcarbonylamino, Alkenylcarbonylamino, gegebenenfalls substituiertes Benzoylamino, gegebenenfalls substituiertes Alkylsulfonylamino, gegebenenfalls substituiertes Phenylsulfonylamino, Alkyl mit 1 bis 4, vorzugsweise 1 oder 2 C-Atomen, Alkoxy mit 1 bis 4, vorzugsweise 1 oder 2 C-Atomen, Chlor oder Brom, Aminosulfonyl, Mono- und Di-alkylaminosulfonyl, deren Alkylreste gegebenenfalls unabhängig voneinander substituiert sein können.

Kupplungskomponenten der Pyridin-Reihe sind zum Beispiel die 6-Hydroxypyridone-2 der Formel II

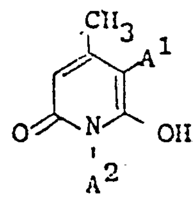

(II)

in der $A^1$ Cyan, Nitro, gegebenenfalls substituiertes Aminocarbonyl, Wasserstoff und $A^2$ Wasserstoff, gegebenenfalls substituiertes Amino oder gegebenenfalls substituiertes Alkyl mit 1 bis 8 C-Atomen bedeuten, ferner die Aminopyridinderivate der Formel III

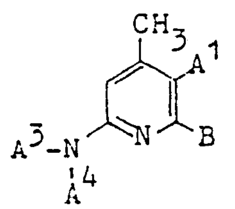

(III)

in der $A^3$ und $A^4$ Wasserstoff oder unabhängig voneinander gegebenenfalls substituiertes Alkyl mit 1 bis 8 C-Atomen, Alkenyl mit 3 bis 5 C-Atomen, Cycloalkyl mit 5 oder 6 C-Atomen und B für $NA^5A^6$ oder für gegebenenfalls substituiertes Alkoxy oder Alkylthio mit 1 bis 8 C-Atomen stehen und $A^5$ und $A^6$ unabhängig voneinander Wasserstoff oder gegebenenfalls substituiertes Alkyl mit 1 bis 8 C-Atomen, Alkenyl mit 3 bis 5 C-Atomen, Cycloalkyl mit 5 oder 6 C-Atomen bedeuten, und $A^1$ die oben genannten Bedeutungen hat, oder die Aminopyridinderivate der Formel IV

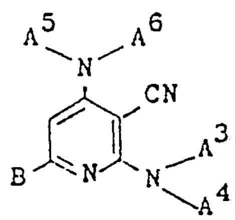

(IV)

in der $A^3$ bis $A^6$ und B die oben genannten Bedeutungen haben. Kupplungskomponenten der Benzpyridin-Reihe sind zum Beispiel : Aminochinoline, N-mono- und N,N-di-Alkyl- oder -Alkenyl-aminochinoline mit 1-8 C-Atomen in den Alkyl- und 3-5 C-Atomen in den Alkenylresten, deren Alkyl- bzw. Alkenylrest gegebenenfalls substituiert sein kann, N-Cycloalkyl- oder N-Phenylaminochinoline, Hydroxychinoline und -isochinoline, N-Alkyl-, Alkenyl- oder -Phenylchinolone und -isochinolone, deren Alkylrest 1-8 C-Atome hat und gegebenenfalls substituiert sein kann. Kupplungskomponenten der Diazol-Reihe sind zum Beispiel : Pyrazolone, Aminopyrazole, N-Alkyl- und N-Phenylpyrazolone bzw. -aminopyrazole, deren Alkylrest 1-8, vorzugsweise 1-4, C-Atome aufweist und deren Alkyl- bzw. Phenylrest gegebenenfalls substituiert sein kann und deren Kern gegebenenfalls durch gegebenenfalls substituiertes Alkyl oder Phenyl, gegebenenfalls substituiertes Alkoxycarbonyl substituiert sein kann.

Kupplungskomponenten aus der Reihe der enolisierbaren 1,3-Dicarbonylverbindungen sind z. B.

gegebenenfalls substituierte Acetoacetyl-arylamide, insbesondere im Kern substituiertes Acetoacetanilid. Als Substituenten kommen in Betracht Chlor, Brom, Methyl, Hydroxy und Methoxy.

Kupplungskomponenten der Thiazol-Reihe sind zum Beispiel : 2-Amino-thiazole, N-mono- und N,N-di-Alkylaminothiazole, deren Alkylreste 1-8 C-Atome aufweisen und gegebenenfalls unabhängig voneinander substituiert sind und deren Kern gegebenenfalls durch gegebenenfalls substituiertes Alkyl mit 1-4 C-Atomen, Alkenyl, Cycloalkyl, Aralkyl, Aryl, Reste heterocyclischer Verbindungen mit aromatischem Charakter, Hydroxy, Amino, Alkylcarbonylamino, Benzoylamino, Alkylsulfonylamino, Phenylsulfonylamino substituiert sein kann.

Kupplungskomponenten der Indol-Reihe sind zum Beispiel : Indol, N-Alkyl-indol, dessen Alkylrest 1-8 C-Atome hat und gegebenenfalls substituiert sein kann und dessen Kern gegebenenfalls durch Alkyl oder Alkoxy mit jeweils 1-4 C-Atomen, Chlor, oder Brom substituiert sein kann.

Kupplungskomponenten der Carbazol-Reihe sind zum Beispiel : Carbazol, N-Alkylcarbazol, dessen Alkylrest 1-8 C-Atome aufweist und gegebenenfalls substituiert sein kann und dessen Kern gegebenenfalls durch Hydroxy, Alkoxy mit 1-4 C-Atomen, Chlor oder Brom substituiert sein kann.

Kupplungskomponenten der Oxazol-Reihe sind zum Beispiel : Hydroxyisooxazole, deren Kern gegebenenfalls durch gegebenenfalls substituiertes Alkyl oder gegebenenfalls substituiertes Alkoxy mit jeweils 1-4 C-Atomen, Phenyl, Chlor oder Brom substituiert sein kann.

Kupplungskomponenten der Diazin-Reihe sind zum Beispiel : N,N-Dialkyl-, N,N-Diaryl- oder N-Alkyl-N-arylbarbitursäuren mit 1-8, vorzugsweise 1-4, C-Atomen in den Alkylresten.

Bevorzugte Anilin-Kupplungskomponenten, die N-monoalkyl-N,N-dialkyl-, N-monoalkenyl-, N,N-dialkenyl-, N-alkyl-N-alkenyl-, N-monocycloalkyl-, N-alkyl-N-cycloalkyl-, N-alkenyl-N-cycloalkyl-, N-phenyl, N-alkyl-N-phenyl-, N-alkenyl-N-phenylsubstituiert sein können, wobei die N-Alkyl-, N-Alkenyl-, N-Cycloalkyl- oder N-Phenylsubstituenten gegebenenfalls substituiert sein können, sind zum Beispiel :

Anilin, 2-Chloranilin, 3-Chloranilin, o- oder m-Toluidin, o-Anisidin, m-Anisidin, 2,5-Dimethoxy- oder -Diethoxyanilin, 2,5-Dimethyl- oder -Diethylanilin, 2-Methoxy- oder -Ethoxy-5-Methyl- oder -5-Chloranilin, 3-(gegebenenfalls substituiertes) Alkyl- oder -Arylcarbonylamino-, 3-(gegebenenfalls substituiertes) Alkyl- oder -Arylsulfonylamino-, 3-(gegebenenfalls substituiertes) Alkoxy- oder -Phenoxycarbonylamino- oder 3-(gegebenenfalls substituiertes) mono- oder -di-Alkylamino-, 3-Phenylamino- oder 3-N-Alkyl-N-phenylaminocarbonylamino-anilin, 2-Chlor-, 2-Brom-, 2-($C_1$ bis $C_4$)-Alkyl- oder 2-(gegebenenfalls substituiertes) Alkoxy-, -5-(gegebenenfalls substituiertes) Alkyl- oder -Arylcarbonylamino-, -5-(gegebenenfalls substituiertes) Alkyl- oder -Arylsulfonylamino-, -5-(gegebenenfalls substituiertes) Alkoxy-, -5-Alkenoxy-, -5-Cycloalkoxy- oder -5-Phenoxycarbonylamino-, -5-(gegebenenfalls substituiertes) mono- oder -di-Alkylamino-, -5-N-Alkyl-N-Phenylamino- oder -5-Phenylaminocarbonylamino-anilin, gegebenenfalls substituiertes Diphenylamin.

Bevorzugte Phenol-Kupplungskomponenten sind zum Beispiel : o-, m-, p-Kresol, 3-Alkyl- oder -Arylcarbonylaminophenol mit gegebenenfalls substituiertem Alkyl- oder Arylrest. Bevorzugte Naphthylamino-Kupplungskomponenten, die durch Alkyl, Alkenyl, Cycloalkyl oder Phenyl N-mono- oder unabhängig voneinander N,N-disubstituiert sein können, wobei die N-Alkyl- und N-Phenylsubstituenten gegebenenfalls substituiert sein können, sind zum Beispiel : Naphthylamin-1, 2-, 5- oder 7-Hydroxy- oder 2-Alkoxynaphthylamin-1, Naphthylamin-2, 5-, 6-, 7- oder 8-Hydroxynaphthylamin-2, 5-Aminosulfonyl-, 5-Mono- oder -N,N-Dialkylaminosulfonyl-naphthylamin-2 mit gegebenenfalls unabhängig voneinander substituiertem Alkylrest.

Bevorzugte Naphthol-Kupplungskomponenten sind zum Beispiel : Naphthol-1, Naphthol-2, 6-Aminosulfonyl-naphthol-2, das durch Alkyl, Alkenyl, Cycloalkyl oder Phenyl unabhängig voneinander N-mono- oder N.N-disubstituiert sein kann und dessen Alkyl- bzw. Phenylreste gegebenenfalls unabhängig voneinander substituiert sein können, 3-Alkoxy- oder Phenoxycarbonylnaphthol-2, 3-Amino-, N-Mono- oder N,N-Dialkylamino- oder -arylaminocarbonylnaphthol-2 mit gegebenenfalls substituiertem N-Alkyl- oder N-Aryl-Rest.

Bevorzugte Kupplungskomponenten der Pyridin-Reihe sind zum Beispiel : 3-Cyano-4-methyl-6-hydroxypyridon-2, N-Alkyl-, N-Alkenyl-, N-Cycloalkyl- oder N-Aryl-3-cyano-4-methyl-6-hydroxypyridone-2, deren Alkyl- oder Arylreste gegebenenfalls substituiert sind, 2.6-Diamino-3-cyan-4-methyl-pyridine, 2.4.6-Triamino-3-cyanopyridine, 2-Amino-6-alkoxy-3- oder -5-cyan-4-methylpyridine, deren Aminogruppen durch gegebenenfalls unabhängig voneinander substituiertes Alkyl oder durch Alkenyl mono- oder disubstituiert bzw. deren Alkoxygruppen gegebenenfalls substituiert sein können.

Bevorzugte Kupplungskomponenten der Benzpyridin-Reihe sind zum Beispiel : 8-Aminochinolin, dessen Aminogruppe durch gegebenenfalls substituiertes Alkyl, durch Alkenyl oder Cycloalkyl mono- oder durch gegebenenfalls unabhängig voneinander substituiertes Alkyl und/oder Alkenyl disubstituiert sein kann, 4-Hydroxychinolin-2 oder 3-Hydroxy-i-chinolon-1, die gegebenenfalls N-mono-alkyl- oder -alkenylsubstituiert sein können, wobei die Alkylreste gegebenenfalls substituiert sein können.

Bevorzugte Kupplungskomponenten der Diazol-Reihe sind zum Beispiel : N-alkyl-, N-alkenyl-, N-cycloalkyl- oder N-arylsubstituierte Pyrazol-3-one, deren N-Alkyl- oder N-Arylrest gegebenenfalls substituiert sein kann und die in 5-Stellung durch gegebenenfalls substituiertes Alkyl, Aminocarbonyl, das durch gegebenenfalls substituiertes Alkyl oder Alkenyl mono- oder disubstituiert sein kann, oder durch gegebenenfalls substituiertes Alkoxy- oder Alkenoxycarbonyl substituiert sein können, oder 3-Amino-5-alkyl-N-arylpyrazole, deren Aminogruppe durch gegebenenfalls substituiertes Alkyl, durch Alkenyl oder

Cycloalkyl mono- oder durch gegebenenfalls unabhängig voneinander substituiertes Alkyl und/oder Alkenyl disubstituiert sein kann.

Bevorzugte Kupplungskomponenten der Thiazol-Reihe sind zum Beispiel : 2-Aminothiazol, dessen Aminogruppe durch gegebenenfalls substituiertes Alkyl, durch Alkenyl oder Cycloalkyl mono- oder durch gegebenenfalls unabhängig voneinander substituiertes Alkyl und/oder Alkenyl disubstituiert sein kann.

Bevorzugte Kupplungskomponenten der Carbazol-Reihe sind zum Beispiel : Carbazol, N-Alkylcarbazol, dessen N-Alkylrest gegebenenfalls substituiert sein kann, N-Alkenylcarbazol, 3-Hydroxycarbazol.

Bevorzugte Kupplungskomponenten der Oxazol-Reihe sind zum Beispiel : 5-Hydroxyisooxazole, die in 3-Stellung durch gegebenenfalls substituiertes Alkyl oder Phenyl, gegebenenfalls substituiertes Alkoxy, Chlor oder Brom substituiert sein können.

Bevorzugte enolisierbare 1,3-Dicarbonylverbindungen sind zum Beispiel : Acetessiganilid und seine N-Chlor-, Brom-, Methyl-, Hydroxy- oder Methoxy-phenyl-Homologe.

Gegebenenfalls substituierte Alkyl-Reste, die in den Substituenten $R^1$, $R^2$ und $A^3$ bis $A^6$ der vorstehend genannten Kupplungskomponenten enthalten sind, sind vor allem lineare oder verzweigte Alkylreste mit 1 bis 8 C-Atomen wie Methyl, Ethyl, n-Propyl, i-Propyl, n-Butyl, i-Butyl, sek. Butyl, n-Pentyl, i-Pentyl, n-Hexyl, i-Octyl, Gegebenenfalls substituierte Alkylcarbonylamino-, Alkylsulfonylamino-, N-Alkyl-N-alkylsulfonylamino-, N-Alkyl-N-phenylsulfonylamino, Alkylaminocarbonylamino- oder Alkoxycarbonylaminogruppen sowie gegebenenfalls substituierte Mono- oder Dialkylaminocarbonyl und Mono- oder Dialkylaminosulfonylgruppen, die als Substituenten in den Kernen von Kupplungskomponenten K stehen können, haben 1-8, vorzugsweise 1-4, C-Atome.

Alkenylreste der vorstehend genannten Kupplungskomponenten sind vor allem solche mit 3 bis 5 C-Atomen wie z. B. Allyl, Methallyl oder Crotyl. Cycloalkyl-Reste der vorstehend genannten Kupplungskomponenten sind vor allem solche mit 5 oder 6 C-Atomen, also Cyclopentyl oder Cyclohexyl, Alkenyl- und Cycloalkylreste der vorstehend genannten Kupplungskomponenten sind vorzugsweise unsubstituiert. Gegebenenfalls substituierte Arylreste der vorstehend genannten Kupplungskomponenten sind vor allem Naphthyl, insbesondere aber Phenylreste.

Die linearen oder verzweigten Alkylreste der Kupplungskomponenten können zum Beispiel folgende Substituenten tragen : Hydroxy ; Alkoxy mit 1 bis 4 C-Atomen ; Alkenyloxy mit 3 bis 5 C-Atomen ; gegebenenfalls substituiertes Phenoxy ; Cycloalkoxy mit 5 oder 6 C-Atomen ; Hydroxyalkoxy, Alkylcarbonyloxyalkoxy oder Phenoxyalkoxy mit 2 bis 4 C-atomen im Alkoxy- bzw. im Alkylcarbonylrest ; Alkoxyalkoxy mit 3 bis 8 C-Atomen ; Hydroxyalkoxyalkoxy, Alkylcarbonyloxyalkoxyalkoxy oder Phenoxyalkoxyalkoxy mit 4 bis 12 C-Atomen im Alkoxyalkoxyrest und 2 bis 4 C-Atomen im Alkylcarbonylrest ; Alkoxyalkoxyalkoxy mit 5 bis 16 C-Atomen ; gegebenenfalls durch Hydroxy, Chlor, Brom, Alkoxy mit 1 bis 4 C-Atomen oder Phenoxy substituiertes Alkylcarbonyloxy mit 2 bis 4 C-Atomen ; gegebenenfalls substituiertes Benzoyloxy ; gegebenenfalls durch Hydroxy, Chlor, Brom, Alkoxy mit 1 bis 4 C-Atomen oder Phenoxy substituiertes Alkoxycarbonyloxy mit 1 bis 4 C-Atomen in der Alkoxygruppe ; Alkenoxycarbonyloxy mit 3 bis 5 C-Atomen in der Alkenoxygruppe ; Cycloalkoxycarbonyloxy mit 5 oder 6 C-Atomen in der Cycloalkoxygruppe ; gegebenenfalls substituiertes Phenoxycarbonyloxy ; gegebenenfalls unabhängig voneinander durch Hydroxy, Chlor, Brom, Alkoxy mit 1 bis 4 C-Atomen oder Phenoxy substituiertes Monoalkylaminocarbonyloxy mit 1 bis 8 C-Atomen in der Alkylaminogruppe ; gegebenenfalls substituiertes Monoarylaminocarbonyloxy ; gegebenenfalls substituiertes Phenyl ; Chlor ; Brom ; Cyan ; und für den Fall, daß R gegebenenfalls substituiertes Alkyl ist, können sie auch folgende Substituenten aufweisen :

Alkoxycarbonyl mit 1 bis 8 C-Atomen in der gegebenenfalls substituierten Alkoxygruppe ; Alkenoxycarbonyl mit 3 bis 5 C-Atomen in der Alkenoxygruppe ; Cycloalkoxycarbonyl mit 5 oder 6 C-Atomen in der Cycloalkoxygruppe ; Phenoxycarbonyl mit gegebenenfalls substituiertem Phenoxyrest.

Die Arylreste, insbesondere die Naphthyl- oder Phenylreste, können insbesondere durch Fluor, Chlor, Brom, Trifluormethyl, Alkyl mit 1 bis 4 C-Atomen, Alkoxy mit 1 bis 4 C-Atomen mono- oder disubstituiert sein.

Besonders bevorzugte Kupplungskomponenten KH sind solche aus der 6-Hydroxypyridon-2-, Aminopyridin-, Naphthol-2, Naphthylamin-1-, 2-Aminothiazol-, Indol- oder Carbazol-Reihe, insbesondere Kupplungskomponenten aus der Anilin-Reihe. Weiterhin sind solche Farbstoffe der Formel I bevorzugt, in denen R für gegebenenfalls substituiertes Alkoxy steht.

Besonders bevorzugte Farbstoffe der Formel I sind solche, die sich aus bevorzugten Diazo- und Kupplungskomponenten zusammensetzen und insbesondere diejenigen, die sich aus besonders bevorzugten Diazo- und Kupplungskomponenten zusammensetzen.

Soll ein ausschließlich hydrophobe Fasern enthaltendes Textilmaterial oder nur der hydrophobe Faseranteil eines Mischtextils coloriert werden, so erfolgt die Durchführung des alkalischen Ätzdruckverfahren in an sich bekannter Weise durch Klotzen der Textilmaterialien mit Farbflotten oder Bedrucken mit Farbpasten, die einen oder mehrere Farbstoffe der Formel I neben den bekannten üblichen Färbereihilfsmitteln, wie beispielsweise Dispergiermitteln, Netzmitteln, Schaumdämpfungsmitteln und Klotzhilfsmitteln bzw. Druckverdickern, enthalten. Geklotzte Gewebebahnen werden auf eine Flottenaufnahme von 50 bis 120 % abgequetscht. Nach diesem ersten Klotz- oder Druckprozess kann die Ware getrocknet oder angetrocknet werden, oder aber es kann ohne jede gesonderte Trocknungsoperation « naß in naß » weitergearbeitet werden. Die Gewebebahnen werden dann mit einer Ätzreservedurckpaste bedruckt, die

als Ätzmittel eine Base, die in 5 %iger wäßriger Lösung einen pH-Wert von mindestens 8 hervorbringt, sowie die in Druckpasten für den Textildruck üblichen bekannten Zusatzstoffe, insbesondere Verdickungsmittel, enthält.

Die oben als erster Verfahrensschritt genannte Operation des Klotzens bzw. Bedruckens mit einer Farbflotte bzw. Farbpaste und das als zweiter Schritt genannte Bedrucken mit der Ätzreservedruckpaste können auch vertauscht werden. In diesem Fall wird das Textilmaterial nach Aufdrucken der Ätzdruckpaste mit der Farbflotte für die Fondfärbung überklotzt bzw. mit der Farbpaste überdruckt. Auch bei dieser Reihenfolge kann « naß in naß » gearbeitet werden oder das Textilmaterial kann nach dem Bedrucken mit der Ätzreservepaste angetrocknet oder getrocknet werden. Anschließend werden die geklotzten und bedruckten Gewebebahnen einer Wärmebehandlung zwischen 100 und 230 °C unterworfen. Im unteren Temperaturbereich von etwa 100 bis 110 °C erfolgt die Wärmezufuhr vorzugsweise durch überhitzten Wasserdampf. Für Wärmebehandlungen, die zwischen 160 und 230 °C durchgeführt werden, wird als Wärmeträger vorzugsweise Heißluft verwendet. Nach der Hitzebehandlung, die eine Fixierung der Dispersionsfarbstoffe sowie die Zerstörung der Farbstoffe der Formel I an den mit der Atzreservedruckpaste bedruckten Stellen zur Folge hat, werden die Textilien in der für Polyester üblichen Art und Weise nachbehandelt, heiß und kalt gespült und getrocknet. Eine besondere Ausführungsform des Ätzdruckverfahrens besteht darin, daß die Klotzflotte außer Farbstoffen der Formel I zusätzlich solche enthält, die alkalibeständig sind und somit durch die einzusetzenden alkalischen Ätzreservedruckpasten nicht zerstört werden. Verfährt man im übrigen wie oben angegeben, so erhält man mehrfarbige Dessins. Wie bereits oben beschrieben besteht eine weitere Ausführungsform des Verfahrens darin, daß man die Farbstoffe der Formel I nicht durch Klotzen mit einer Klotzflotte auf dem gesamten Gewebe appliziert, sondern ebenfalls in Form von Druckpasten auf das Gewebe aufdruckt.

Fixierung und Fertigstellung der Textildrucke erfolgt dann anschließend wie oben bereits beschrieben. Auch bei diesem Verfahren ist es möglich, den aufgedruckten Farbdruckpasten Farbstoffe zuzusetzen, die alkaliresistent sind. Auch in diesem Falle werden mehrfarbige Dessins erhalten.

Eine weitere Möglichkeit zur Durchführung des erfindungsgemäßen Verfahrens besteht darin, daß auf den mit Farbstoffen der Formel I geklotzten oder bedruckten Fond Ätzreservedruckpasten aufgedruckt werden, die ihrerseits alkaliresistente Farbstoffe enthalten. Bei anschließender Fixierung und Fertigstellung der Textilmaterialien, wie oben beschrieben, werden auch hier mehrfarbige Dessins erhalten.

Die Farbstoffe der Formel I liegen in den Klotzflotten bzw. in den Druckpasten in fein dispergierter Form vor, wie es für Dispersionsfarbstoffe üblich und bekannt ist. Auch die Herstellung der Klotzflotten bzw. Druckpasten, die erfindungsgemäß einzusetzen sind, erfolgt in an sich bekannter Weise durch Mischen der Flotten- bzw. Druckpastenbestandteile mit der nötigen Menge Wasser und flüssigen feindispersen oder festen redispergierbaren Einstellungen der Farbstoffe der Formel I.

Alkaliresistente Dispersionsfarbstoffe, die zur Herstellung von mehrfarbigen Dessins mit dem Farbstoff der Formel I kombiniert werden können, sind die bekannten Handelsfarbstoffe aus der Gruppe der Azo- oder Azomethin-, Chinophthalon-, Nitro- oder Anthrachinonfarbstoffe. Einige Beispiele für alkaliresistente Dispersionsfarbstoffe sind :

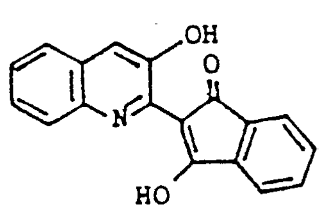 , 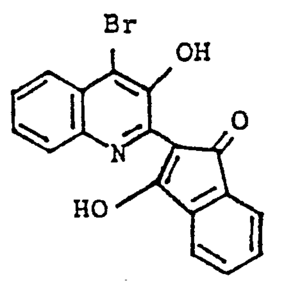

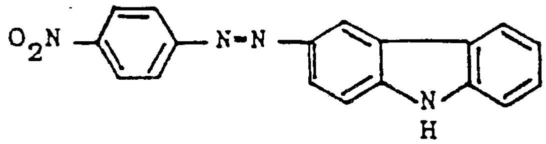 , 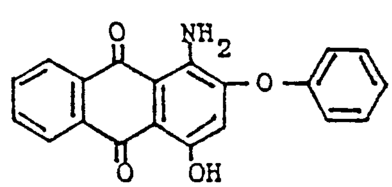

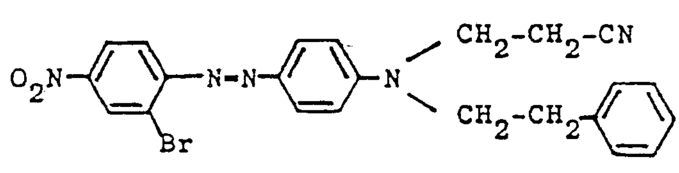

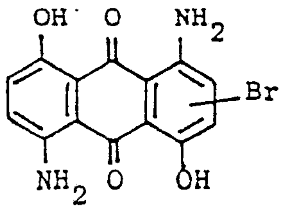 , 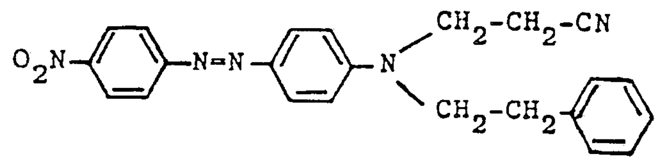

Basen, die als Ätzmittel in der Ätzreservedruckpaste enthalten sind und die in 5 %iger wäßriger Lösung mindestens einen pH-Wert von 8 hervorbringen, sind in großer Zahl bekannt. Beispiele für solche Basen sind die Hydroxide der Alkali- und Erdalkalimetalle, Salze von Erdalkali- und Alkalimetallen mit schwachen organischen oder anorganischen Säuren, wie z. B. Alkaliacetat, Alkalicarbonate oder -bicarbonate, Trialkaliphosphate, Ammoniak oder auch aliphatische Amine, wie z. B. Triethyl-, Tripropyl- oder Tributylamin, Ethanolamin, Dimethyl- oder Diethylethanolamin, Diethanolamin, Methyl-, Ethyl- oder Propyl-diethanolamin oder Triethanolamin. Üblicherweise werden als Basen Erdalkalihydroxide, wie z. B. Calciumhydroxid, Alkalihydroxide, wie beispielsweise Natrium- oder Kaliumhydroxid, oder Alkalisalze von schwachen anorganischen Säuren, wie beispielsweise Natriumcarbonat, Trinatriumphosphat oder Natrium- bzw. Kaliumsilicat, eingesetzt. Vorzugsweise wird als Base in den Ätzreservedruckpasten Natrium- oder Kaliumhydroxid oder insbesondere Natrium- oder Kaliumcarbonat oder Natrium- oder Kaliumbicarbonat verwendet. Auch Mischungen verschiedener Basen können verwendet werden. Die Konzentration der Base in den Ätzreservedruckpasten beträgt zweckmäßigerweise 25 bis 250 g/kg, vorzugsweise 50 bis 130 g/kg. Die Ätzreservedruckpasten enthalten neben den genannten Basen die üblichen, in Textildruckpasten enthaltenen Zusätze, insbesondere Verdikkungsmittel, wie z. B. Alginate, Stärkeprodukte, synthetische polymere Verdickungsmittel, Mineralöle, hydrotrope Substanzen, wie beispielsweise Harnstoff, sowie Zusätze, welche die Benetzung, Durchdringung und Farbstoffaufnahme fördern. Besonders günstig für den Ätzvorgang ist die Anwesenheit nichtionogener Detergenzien, die zweckmäßigerweise in den Ätzreservedruckpasten enthalten sind, wie z. B. Glycerin und/oder Polyglykole, wie Polyäthylenglykol mit einem mittleren Molekulargewicht von 300 bis 400.

Nach dem beschriebenen alkalischen Ätzdruckverfahren lassen sich Ätzreservedrucke nicht nur auf Textilmaterialien aufbringen, die aus hydrophoben Fasern, insbesondere Polyesterfasern, bestehen oder solche Fasern überwiegend enthalten, sondern auch auf Textilmaterialien, die hydrophobe Fasern, insbesondere Polyesterfasern, und Cellulosefasern in vergleichbaren Mengenverhältnissen enthalten. Derartige Polyester/Cellulose-Mischgewebe können z. B. ein Gewichtsverhältnis Polyester/Cellulose von 75 : 25, 65 : 35 oder 50 : 50 aufweisen. Das Aufbringen von Ätzreservedrucken auf derartige Mischgewebe nach dem genannten Verfahren ist dann möglich, wenn die Farbflotte oder Druckpaste, welche mindestens einen weißätzbaren Dispersionsfarbstoff der Formel I und gegebenenfalls noch einen oder mehrere ätzmittelbeständige Dispersionsfarbstoffe enthält, außerdem noch mindestens einen ätzbaren Reaktivfarbstoff mit einem reaktiven Rest der Formel

$—SO_2—CH_2—CH_2—Hal$ oder
$—SO_2—CH_2—CH_2—O—SO_3X$ oder
$—NH—SO_2—CH_2—CH_2—OSO_3X$ oder
$—SO_2—CH=CH_2$

worin X Wasserstoff oder ein Metallkation, insbesondere das Natriumkation, und Hal Halogen, insbesondere Chlor oder Brom, bedeuten, und gegebenenfalls einen oder mehrere ätzbeständige Reaktivfarbstoffe enthält und wenn die Ätzreservedruckpaste neben Alkalicarbonat oder Alkalihydrogencarbonat ein Alkalisulfit oder Alkalihydrogensulfit und gegebenenfalls einen Aldehyd enthält und wenn im übrigen wie bereits angegeben gearbeitet wird.

Die einzusetzenden ätzbaren Reaktivfarbstoffe enthalten einen der oben angegebenen fasserreaktiven Reste. Diesen Resten ist es gemeinsam, daß sie in Gegenwart von Alkali unter Abspaltung eines Sulfat- oder Halogenidanions eine Vinylsulfonylgruppe ausbilden. Diese in Gegenwart von Alkali gebildete Gruppe fixiert auf Baum- oder Zellwolle in gleicher Weise wie der direkt an den Farbstoffrest gebundene Vinylsulfonylrest der Formel VII durch Addition einer OH-Gruppe der Cellulose an die Vinyldoppelbindung. Ätzbare Reaktivfarbstoffe, die einen der oben genannten reaktiven Reste aufweisen, können allen technisch wichtigen Farbstoffgruppen angehören. Als Beispiele für geeignete Reaktivfarbstoffe werden die Monoazofarbstoffe CI Yellow 13 bis 17 und 72 bis 74, Orange 7, 15, 16, 23, 24, 55, Red 21 bis 23, 35, 36, 50, 63, 103 bis 107, 112 bis 114, Blue 28, Brown 16 ; die Diazofarbstoffe CI Blue 76, Blue 98, Black 5, 31 ; die Mono- bzw. Disazo-Metallkomplex-Farbstoffe CI Violet 4, 5, Blue 20, Brown 18 ; die Anthrachinonfarbstoffe CI Violet 22, Blue 19 und 27 ; die Phthalocyaninfarbstoffe CI Blue 21, 38, 77, 91 und Green 14 genannt.

Die Dispersionsfarbstoffe der Formel I liegen in den Klotzflotten bzw. in den Druckpasten in fein dispergierter Form vor, wie es für Dispersionsfarbstoffe üblich und bekannt ist, während die Reaktivfarbstoffe gelöst sind. Auch die Herstellung der Klotzflotten bzw. Druckpasten, die erfindungsgemäß einzusetzen sind, erfolgt in an sich bekannter Weise durch Mischen der Flotten- bzw. Druckpastenbestandteile mit der nötigen Menge Wasser und flüssigen feindispersen oder festen redispergierbaren Einstellungen der Dispersionsfarbstoffe sowie Lösungen bzw. Einstellungen der Reaktivfarbstoffe.

Wie beim Verfahren der DE-A-30 19 726 ist es auch hier besonders bei der Herstellung von Buntätzen vorteilhaft, die in den Reservepasten neben den genannten Reservierungsmitteln üblichen Zusätze, insbesondere Verdickungsmittel wie z. B. Alginate, Stärkeprodukte, synthetische polymere Verdickungsmittel, Mineralöle, hydrotrope Substanzen, wie beispielsweise Harnstoff, sowie Benetzung, Durchdringung und Farbstoffaufnahme fördernde Zusätze, nichtionogene Detergentien, wie z. B. Glycerin, und/oder Polyglykole, wie Polyäthylenglykol mit einem mittleren Molekulargewicht von 300 bis 400, durch Ester höhermolekularer Polyglykole mit Carbonsäuren zu ersetzen.

Polyglykolcarbonsäureester, die mit besonderem Vorteil in den erfindungsgemäß zu verwendenden Reservepasten eingesetzt werden können, sind beispielsweise in der Deutschen Auslegeschrift 11 38 735 beschrieben.

Besonders vorteilhaft ist es auch hier, gemäß der DE-A-29 52 312 bei der Herstellung der Druckpaste auf den üblichen Zusatz von Mineralölen zu verzichten und statt dessen Druckpasten einzusetzen, die pro kg 30 bis 250 g, vorzugsweise 50 bis 150 g, eines Hilfsmittels der allgemeinen Formel VIII

$$C_nH_{2n+2-m}[O(CH_2—CH(CH_3)O)_{x/m}—H]_m \quad (VIII)$$

wobei n eine ganze Zahl von 2 bis 6, m eine ganze Zahl von 1 bis 6 und x eine Zahl von 20 bis 60 ist mit der Maßgabe, daß m kleiner oder gleich n ist, gegebenenfalls in Kombination mit einem Veresterungsprodukt von höhermolekularen Polyglykolen mit höheren Alkan- und Alkencarbonsäuren enthalten.

Die Farbstoffe der allgemeinen Formel I können hergestellt werden, indem man eine Diazokomponente der Formel V

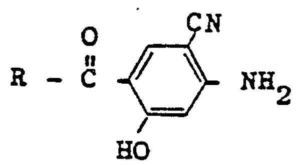 (V)

diazotiert und auf eine Kupplungskomponente der Formel VI

$$K—H \quad (VI)$$

kuppelt.

Die Diazotierung des Amins der Formel V geschieht in an sich bekannter Weise durch Einwirkung von salpetriger Säure oder salpetrige Säure abspaltender Verbindungen. Beispielsweise können die Amine in Schwefelsäure, Salzsäure, Phosphorsäure oder in niederen aliphatischen Carbonsäuren, wie z. B. Essigsäure oder Propionsäure, gelöst werden und bei 0 bis 60 °C durch Zusatz von Nitrosylschwefelsäure bzw. Natriumnitrit diazotiert werden. Die Kupplung auf eine am Kern aminogruppenhaltige Kupplungskomponente der allgemeinen Formel VI wird z. B. im salz- oder schwefelsauren wäßrigen Medium, in einer niederen aliphatischen Carbonsäure, wie z. B. Essigsäure, die gegebenenfalls mit Wasser verdünnt ist, bzw. in einer Mischung aus Wasser und einem in Wasser wenig löslichen Alkohol, wie n- oder i-Butanol, bei Temperaturen von 0 bis 50 °C ausgeführt. Bevorzugt wird hierbei der Temperaturbereich von 0 bis 30 °C. Zur Vervollständigung der Kupplungsreaktion kann es zweckmäßig sein, den pH-Wert des Kupplungsansatzes am Anfang oder gegen Ende der Reaktion durch Zusatz von Basen, wie z. B. von Natriumacetat, auf einen Wert von 3 bis 6 zu puffern.

Bei der Kupplung auf eine am Kern hydroxygruppenhaltige Kupplungskomponente der allgemeinen Formel VI wird im wäßrigen Medium oder in einer Mischung aus Wasser und einem in Wasser wenig löslichen Alkohol, um die Geschwindigkeit der Reaktion zu erhöhen, diese Kupplungskomponente zusammen mit einer Base wie Natron- oder Kalilauge, Soda, Pottasche oder Natriumhydrogencarbonat vorgelegt und das Reaktionsmedium durch Zugabe von z. B. Natriumacetat gepuffert. Die Isolierung der Farbstoffe erfolgt in der üblichen Art und Weise durch Filtration.

Die Farbstoffe der allgemeinen Formel I können auch aus Farbstoffen, die anstelle der in der Diazokomponente enthaltenen Cyangruppe ein zur Azobrücke o-ständiges Chlor- oder Bromatom enthalten, durch Austausch des Chlors oder Broms gegen Cyan nach Austauschverfahren hergestellt werdan, wie sie z. B. in den Deutschen Offenlegungsschriften 15 44 563, 23 10 745, 24 56 495, 26 10 675, 27 24 116, 27 24 117, 28 34 137, 28 34 386, 28 46 438, 29 15 072 und 29 31 081 beschrieben sind.

Das Amin der Formel V, wobei R Ethoxy bedeutet, ist in Liebigs Ann. Chem. *1979,* 2005 bis 2010, beschrieben. Es wird nach einem dort beschriebenen Verfahren durch Kondensation eines β-Oxocarbonsäureesters mit Ethoxymethylenmalodinitril in Ethanol in Gegenwart von Natriumethylat oder durch Kondensation von Ethoxymethylenacetessigester und Malodinitril ebenfalls in Ethanol in Gegenwart von Natriumethylat und anschließendes Ansäuern erhalten. Analog lassen sich bei Verwendung

entprechend substituierter β-Oxocarbonsäureester Amine der Formel V herstellen, in denen R ein gegebenenfalls substituierter Alkoxyrest der oben gegebenen Definition ist.

Amine der allgemeinen Formel V, in der R ein gegebenenfalls substituierter Alkylrest ist, können nach der Arbeitsvorschrift in J. Chem. Soc. Perkin I (1979), S. 684 hergestellt werden.

Nach diesen Verfahren können beispielsweise die in der folgenden Liste angegebenen Diazokomponenten der allgemeinen Formel V hergestellt werden, die zur Herstellung von Farbstoffen der allgemeinen Formel I eingesetzt werden können :

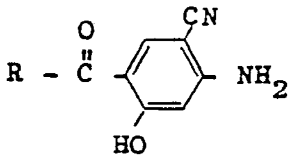

(V)

R:

$-CH_3$ ; $-C_2H_5$ ; $-(n)C_3H_7$ ; $-(i)C_3H_7$ ; $-(n)C_4H_9$ ; $-(i)C_4H_9$ ;
$-(n)C_5H_{11}$ ; $-(i)C_5H_{11}$ ; $-(sec.)C_5H_{11}$ ; $-(i)C_7H_{15}$ ;
$-CH_2CH(C_2H_5)C_4H_9$ ; $-CH_2OH$ ; $-CH_2Cl$ ; $-CH_2Br$ ; $-CH_2-OCH_3$ ;
$-CH_2-OC_2H_5$ ; $-CH_2-O-$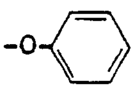 ; $-CH_2-OCH_2-CH{=}CH_2$ ; $-CH_2-$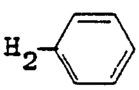 ;
$-CH_2-O-$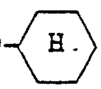 ; $-CH_2-O(i)C_3H_7$ ; $-CH_2-O-COCH_3$ ; $-CH_2-O-COC_2H_5$;
$-(CH_2)_2OH$ ; $-(CH_2)_2Br$ ; $-(CH_2)_2-OCH_3$ ; $-(CH_2)_2-O-$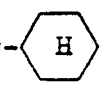 ;
$-(CH_2)_2-O(iso)C_3H_7$ ; $-CH_2-O(CH_2)_2-OC_2H_5$ ; $-(CH_2)_3-OH$ ;
$-(CH_2)_3Cl$ ; $-(CH_2)_3-OCH_3$ ; $-(CH_2)_3-OC_2H_5$ ;
$-(CH_2)_3-OC_2H_4-OCH_3$ ; $-(CH_2)_2-O-$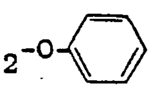 ; $-(CH_2)_2-O-COCH_3$ ;
$-CH_2CH(OH)CH_2Cl$, $-O-C_2H_5$ ; $-O-C_2H_5$ ; $-O-C_2H_5$ ; $-O-CH_3$ ;
$-O-C_2H_5$ ; $-O-C_2H_5$ ; $-O-C_2H_5$ ; $-O-CH_3$ ; $-O-CH_3$ ; $-O-CH_3$ ;
$n-C_3H_7-O-$ ; $i-C_3H_7-O-$ ; $n-C_4H_9-O-$ ; $i-C_7H_{15}-O-$ ;
$-O-(CH_2)_2OCH_3$ ; $-O-C_6H_5$ ; $i-C_4H_9-O-$ ; $-O-(CH_2)_2O(CH_2)_2OCH_3$ ;
$-O-(CH_2)_2OC_6H_5$ ; $-O-CH_2CH(OH)CH_2Cl$ ; $-O-CH_2C_6H_5$ ;
$-O-(CH_2)_2OCOCH_3$ ; $-O-CH_2CH(C_2H_5)C_4H_9$ ; $-O-(CH_2)_2OC_6H_{11}$ ;
$-O-(CH_2)_2Oi-C_3H_7$ ; $-O-(CH_2)_2OH$ ; $-O-(CH_2)_2CN$.

In den bei obigen Applikationen eingesetzten Klotzflotten und Druckpasten liegen die erfindungsgemäßen Farbstoffe, wie bei Dispersionsfarbstoffen üblich, in möglichst feiner Verteilung vor.

Die Feinverteilung der Farbstoffe erfolgt in an sich bekannter Weise dadurch, daß man den in der Fabrikation anfallenden Farbstoff zusammen mit Dispergiermitteln in einem flüssigen Medium, vorzugsweise in Wasser, aufschlämmt und die Mischung der Einwirkung von Scherkräften aussetzt, wobei die ursprünglich vorhandenen Farbstoff-Teilchen mechanisch so weit zerkleinert werden, daß eine optimale spezifische Oberfläche erreicht wird und die Sedimentation des Farbstoffs möglichst gering ist. Die Teilchengrößen der Farbstoffe liegen im allgemeinen zwischen 0,5 und 5 µm, vorzugsweise bei etwa 1 µm.

Die bei dem Mahlvorgang mitverwendeten Dispergiermittel können nichtionogen oder anionaktiv sein. Nichtionogene Dispergiermittel sind z. B. Umsetzungsprodukte von Alkylenoxiden wie z. B. Ethylen- oder Propylenoxid mit alkylierbaren Verbindungen wie z. B. Fettalkoholen, Fettaminen, Fettsäuren, Phenolen, Alkylphenolen und Carbonsäure-amiden. Anionaktive Dispergiermittel sind beispielsweise Ligninsulfonate, Alkyl- oder Alkylarylsulfonate oder Alkyl-aryl-polyglycoläthersulfate.

Das erfindungsgemäße Verfahren liefert Ätzdrucke von hoher Brillanz mit scharf stehenden Konturen und einwandfreiem Weißfond. Es ist auch möglich, nach dem erfindungsgemäßen Verfahren Buntdrucke herzustellen, die sich durch Exaktheit der Detailwiedergabe und Farbreinheit auszeichnen. Das Echtheitsniveau der erfindungsgemäß hergestellten Ätzdrucke ist ausgezeichnet insbesondere auch bei Drucken auf Polyester-Zellulose Mischgeweben. Insbesondere sind die Wasch-, Reib-, Trockenreinigungs-, Trockenhitzefixier- und Lichtechtheit hervorzuheben, die in Kombination mit der durch das schonende erfindungsgemäße Verfahren nicht verminderten mechanischen Festigkeit der Gewebe den hohen Gebrauchswert der hergestellten Ätzdrucke begründen.

Besonders überraschend war es, daß die erfindungsgemäß einzusetzenden Farbstoffe sich durch ein so schonendes Ätzmittel rein weiß ätzen lassen. Bisher ist man nämlich der Auffassung gewesen, daß diese Eigenschaft nur dann gegeben ist, wenn im Farbstoffmolekül mindestens zwei leicht verseifbare Karbonestergruppen enthalten sind, oder wenn Farbstoffe eingesetzt werden, deren Chromophor durch Alkali angegriffen wird, wie z. B. bei den Farbstoffen der DE-A-2 836 391.

Die folgenden Ausführungsbeispiele veranschaulichen die Durchführung des erfindungsgemäßen Verfahrens und die Herstellung der dabei eingesetzten Farbstoffe. %-Angaben beziehen sich auf Gewicht.

Beispiel 1

a) 10 g des Farbstoffs der Formel

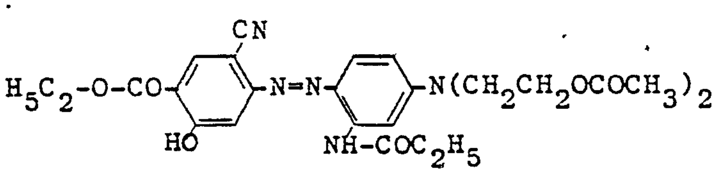

werden in feindispergierter Form zu einer Klotzflotte gegeben, die 905 Teile Wasser, 5 Teile Citronensäure und 60 Teile eines Polymerisationsprodukts auf Acrylsäurebasis als Antimigrationsmittel auf 1 000 Teile enthält. Mit dieser Klotzflotte wird ein Gewebe aus Polyester (PES) auf der Basis von Polyethylenglykolterephthalat bei 20 bis 30 °C mit einem Abquetscheffekt von ca. 80 % geklotzt. Das geklotzte Gewebe wird bei 60 bis 80 °C vorsichtig getrocknet. Nach dem Trocknen wird mit einer Druckpaste, die 500 Teile einer wäßrigen 10 %igen Johannisbrotkernmehlätherverdickung, 260 Teile Wasser, 80 Teile calciniertes Natriumcarbonat, 80 Teile Polyethylenglykol 400 und 80 Teile Glycerin auf 1 000 Teile enthält, überdruckt. Nach dem Fixieren mit überhitztem Dampf während 7 Minuten bei 175 °C, reduktivem Nachbehandeln mit einer 0,2 %igen Natriumdithionitlösung während 15 Minuten bei 70 bis 80 °C, Seifen, anschließendem Spülen und Trocknen erhält man einen roten Druck mit sehr guten Echtheiten, vor allem guter Licht-, Trockenhitzefixier-, Reib- und Waschechtheit. An den Stellen auf die sodahaltige Druckpaste aufgedruckt wird, erhält man einen sehr guten Weißfond mit scharfen Konturen.

b) Der im Abschnitt a) eingesetzte Farbstoff wird wie folgt hergestellt:
20,6 g des Amins der Formel

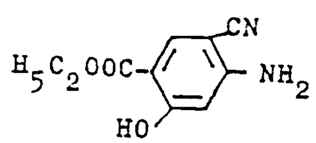

werden in 125 ml Eisessig bei 15 bis 20 °C mit 34,2 g 40,5 %iger Nitrosylschwefelsäure diazotiert und bei 0-5 °C auf eine Lösung von 35,3 g des Amins der Formel

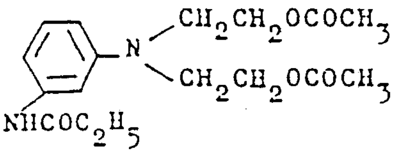

in 450 ml 50 %iger Essigsäure in Gegenwart von 1 g Amidosulfosäure gekuppelt. Es wird 1 Stunde bei dieser Temperatur nachgerührt, der Farbstoff durch langsame Zugabe von 500 ml Eiswasser gefällt, abfiltriert, mit Wasser gewaschen und unter vermindertem Druck getrocknet. Es werden 50,5 g des Farbstoffs der oben angegebenen Formel vom Schmelzpunkt 153-155 °C erhalten, der sich mit roter Farbe in o-Dichlorbenzol löst.

Beispiel 2

Man klotzt ein mercerisiertes Mischgewebe aus Polyester-Baumwolle 65 : 35 mit einem Ansatz bestehend aus 100 Gewichtsteilen einer 10 %igen Flüssigeinstellung des Farbstoffs der in Beispiel 1, Abschnitt a) angegebenen Formel, 40 Gewichtsteilen der flüssigen Handelsform von C.I. Reactiv Orange 16, 808 Gewichtsteilen Wasser kalt, 10 Gewichtsteilen m-nitrobenzolsulfonsaurem Natrium, 20 Gewichtsteilen eines Antimigriermittels auf Polyacrylsäurebasis, 2 Gewichtsteilen Mononatriumphosphat, 20 Gewichtsteilen Natriumformiat.

Man trocknet vorsichtig in einer Hotflue bei 80-100 °C und überdruckt im Filmdruck mit einer Druckpaste aus 25 Gewichtsteilen der Handelsform von [5-(3,6-Dichlorpyridazin-4-yl-carbonylamino)-2-methyl-3-sulfophenyl]-3-carboxy-4-(2-sulfophenylazo)-5-pyrazolon-(5), 40 Gewichtsteilen der flüssigen Handelsform von C.I. Disperse Yellow 63, 150 Gewichtsteilen Harnstoff, 199 Gewichtsteilen Wasser kalt, 10 Gewichtsteilen m-nitrobenzolsulfonsaurem Natrium, 500 Gewichtsteilen Stammverdickung, die sich aus 230 Gewichtsteilen einer wäßrigen 4 %igen Alginatverdickung, 80 Gewichtsteilen einer wäßrigen 10 %igen Stärkeätherverdickung, 85 Gewichtsteilen Wasser, 25 Gewichtsteilen einer wäßrigen 10 %igen Lösung des Kondensationsproduktes von Polyglykol 2 000 mit Stearinsäure und 80 Gewichtsteilen Schwerbenzin zusammensetzt, 40 Gewichtsteilen Natriumhydrogencarbonat, 30 Gewichtsteilen Natriumhydrogensulfitlösung 38°Bé, 6 Gewichtsteilen 40 %iger Glyoxallösung.

Nach dem Trocknen wird 7 Minuten bei 175 °C mit überhitztem Wasserdampf fixiert und wie im Beispiel 1 angegeben nachbehandelt.

Analog den Angaben in den Beispielen 1 und 2 können auch die Farbstoffe des folgenden Herstellungsbeispiels und der anschließenden Tabelle erfindungsgemäß eingesetzt werden.

Beispiel 3

20,6 des wie in Beispiel 1 diazotierten Amins werden bei 0-10 °C innerhalb ca. einer halben Stunde auf eine Lösung von 29,2 g des Amins der Formel

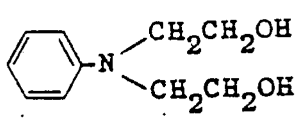

in 700 ml 75 %iger Essigsäure in Gegenwart von 66 g wasserfreiem Natriumacetat gekuppelt. Der Ansatz wird ca. 3 Stunden unter Erwärmung auf Raumtemperatur nachgerührt, dann wird der ausgefallene Farbstoff abgesaugt, mit Wasser gewaschen und unter vermindertem Druck getrocknet. Es werden 17,5 g Farbstoff der Formel

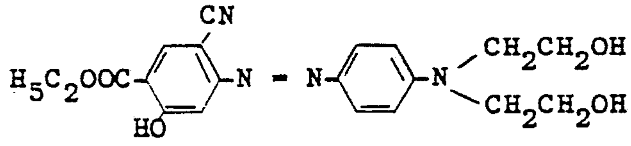

vom Schmelzpunkt 127-129 °C erhalten, der sich mit oranger Farbe in o-Dichlorbenzol löst.

(Siehe Tabellen Seite 14 ff.)

Tabelle I

Farbstoffe der Formel

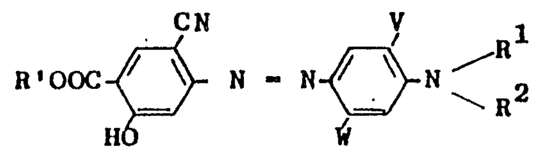

| R' | V | W | $R^1$ | $R^2$ | Farbe auf PES |
|---|---|---|---|---|---|
| $C_2H_5$ | H | H | $(CH_2)_2OCOCH_3$ | $(CH_2)_2CN$ | Orange |
| $C_2H_5$ | H | H | $(CH_2)_2C_6H_5$ | $(CH_2)_2CN$ | orange |
| $C_2H_5$ | H | H | $C_2H_5$ | $(CH_2)_2OH$ | orange |
| $CH_3$ | H | H | $C_2H_5$ | $(CH_2)_2OCOCH_3$ | orange |
| $C_2H_5$ | H | H | $(CH_2)_2OH$ | $(CH_2)_2CN$ | orange |
| $CH_3$ | H | H | $CH(CH_3)_2CN$ | H | orange |
| $n-C_3H_7$ | H | H | $CH(C_2H_5)_2$ | H | orange |
| $n-C_4H_9$ | H | H | $(CH_2)_2OCOOCH_3$ | $CH_2CH-CH_3$ | orange |
| $(CH_2)_2OCH_3$ | H | H | $CH_3O(CH_2)_2$ | $CH_3O(CH_2)_2$ | orange |
| $C_2H_5$ | Cl | H | $(CH_2)_2CN'$ | H | orange |
| $CH_3$ | Cl | H | $(CH_2)_2O(CH_2)_2OCH_3$ | H | orange |
| $C_2H_5$ | $CH_3$ | H | $CH(CH_3)_2$ | H | orange |

Tabelle I (Fortsetzung)

| R' | V | W | R¹ | R² | Farbe auf PES |
|---|---|---|---|---|---|
| $C_6H_5$ | $CH_3$ | H | $(CH_2)_2OC_6H_5$ | H | orange |
| $C_2H_5$ | H | $CH_3$ | $C_2H_5$ | $C_2H_5$ | orange |
| $(CH_2)_2OC_6H_5$ | H | $CH_3$ | $(CH_2)_2OCOCH_3$ | $(CH_2)_2OCOCH_3$ | orange |
| $CH_3$ | H | $CH_3$ | $(CH_2)_2CN$ | $n\text{-}C_3H_7$ | orange |
| $i\text{-}C_3H_7$ | H | $CH_3$ | $(CH_2)_2CN$ | $(CH_2)_2OH$ | orange |
| $C_2H_5$ | H | $CH_3$ | $n\text{-}C_4H_9$ | $(CH_2)_2CN$ | orange |
| $C_2H_5$ | H | $CH_3$ | $COCH_2OCH_3$ | H | goldgelb |
| $(CH_2)_2O(CH_2)_2OC_2H_5$ | H | $CH_3$ | $(CH_2)_2Cl$ | $(CH_2)_2Cl$ | orange |
| $CH_3$ | H | $CH_3$ | $(CH_2)_2CN$ | $(CH_2)_2OCOC_2H_5$ | orange |
| $(CH_2)_2CN$ | H | H | $C_6H_5$ | H | orange |
| $C_2H_5$ | $OCH_3$ | H | $(CH_2)_2OCOC_3H_7$ | $(CH_2)_2OCOC_3H_7$ | orange |
| $CH_3$ | $OCH_3$ | H | $CH(CH_3)$ | H | orange |
| $(CH_2)_2OH$ | H | $OCH_3$ | $n\text{-}C_3H_7$ | $n\text{-}C_3H_7$ | orange |
| $C_2H_5$ | $OCH_3$ | $OCH_3$ | $C_2H_5$ | $C_2H_5$ | scharlach |
| $C_2H_5$ | $OCH_3$ | $OCH_3$ | $CH_2CH(OH)CH_2OC_6H_5$ | H | scharlach |
| $CH_3$ | $OCH_3$ | $CH_3$ | $n\text{-}C_4H_9$ | $n\text{-}C_4H_9$ | scharlach |

Tabelle I (Fortsetzung)

| R' | V | W | $R^1$ | $R^2$ | Farbe auf PES |
|---|---|---|---|---|---|
| $CH_3$ | $CH_3$ | $CH_3$ | $(CH_2)_2CN$ | $(CH_2)_2OH$ | scharlach |
| $i\text{-}C_4H_9$ | $OCH_3$ | Cl | $(CH_2)_2C_6H_5$ | $(CH_2)_2CN$ | scharlach |
| $C_2H_5$ | $CH_3$ | $CH_3$ | $(CH_2)_2OH$ | $(CH_2)_2OH$ | scharlach |
| $(i)C_3H_7$ | $OCH_3$ | $CH_3$ | $CH(CH_3)C_2H_5$ | $(CH_2)_2OH$ | scharlach |
| $CH_3$ | $CH_3$ | Cl | $CH(CH_2OH)C_6H_5$ | H | rot |
| $i\text{-}C_3H_7$ | H | $NHCOCH_3$ | $C_2H_5$ | $(CH_2)_2CN$ | rot |
| $C_2H_5$ | H | $NHCOCH_3$ | $(CH_2)_2CN$ | $CH_2CH{=}CH_2$ | rot |
| $(CH_2)_2OC_6H_5$ | H | $NHCOCH_3$ | $(CH_2)_2CN$ | $(CH_2)_2OH$ | rot |
| $CH_3$ | H | $NHCOC_2H_5$ | $C_2H_5$ | $C_2H_5$ | rot |
| $CH_3$ | H | $NHCOC_2H_5$ | $(CH_2)_2CN$ | $(CH_2)_2OCOCH_3$ | rot |
| $C_2H_5$ | H | $NHCOC_2H_5$ | $[(CH_2)_2O]_2H$ | $[(CH_2)_2O]_2H$ | rot |
| $C_2H_5$ | H | $NHCOC_2H_5$ | $CH_2CH(OH)CH_2Cl$ | $CH_2CH(OH)CH_2Cl$ | orange |
| $(i)C_3H_7$ | H | $NHCO\text{-}C_4H_9(n)$ | $C_2H_5$ | $C_2H_5$ | orange |
| $C_2H_5$ | H | $NHCOC_6H_5$ | $CH_2C_6H_5$ | H | scharlach |
| $[(CH_2)_2O]_2CH_3$ | H | $NHSO_2CH_3$ | $(CH_2)_2OH$ | $(CH_2)_2OH$ | rot |

Tabelle I (Fortsetzung)

| R' | V | W | $R^1$ | $R^2$ | Farbe auf PES |
|---|---|---|---|---|---|
| $C_2H_5$ | Cl | $NHCOCH_3$ | $CH(C_2H_5)_2$ | H | rot |
| $C_2H_5$ | Cl | $NHCOCH_3$ | $CH_2CH{=}CH_2$ | $CH_2CH{=}CH_2$ | rot |
| $CH_3$ | $CH_3$ | $NHCOCH_3$ | $(CH_2)_2CN$ | H | rot |
| $C_2H_5$ | $CH_3$ | $NHCOCH_3$ | $(CH_2)_2CH$ | $CH_2CH{=}CH_2$ | rubin |
| $n\text{-}C_4H_9O(CH_2)_2$ | $CH_3$ | $NHCOCH_2OCH_3$ | $C_2H_5$ | $C_2H_5$ | rubin |
| $i\text{-}C_3H_7$ | $OCH_3$ | $NHCOCH_3$ | $(CH_2)_2OCOCH_3$ | $(CH_2)_2OCOCH_3$ | violett |
| $CH_3$ | $OCH_3$ | $NHCOCH_3$ | $(CH_2)_2CN$ | $CH_2CH{=}CH_2$ | violett |
| $C_2H_5$ | $OC_2H_5$ | $NHCOCH_3$ | $(CH_2)_2OH$ | $(CH_2)_2OH$ | violett |
| $C_2H_5$ | $OC_2H_5$ | $NHCOCH_3$ | $(CH_2)_2OCOCH_3$ | $(CH_2)_2OCOCH_3$ | violett |
| $CH_3$ | $O(CH_2)_2OCH_3$ | $NHCOC_2H_5$ | $C_2H_5$ | H | violett |
| $CH_3$ | $O(CH_2)_2OH$ | $NHCOCH_3$ | $CH(CH_3)C_2H_5$ | H | violett |

Tabelle I (Fortsetzung)

Farbstoffe der Formel

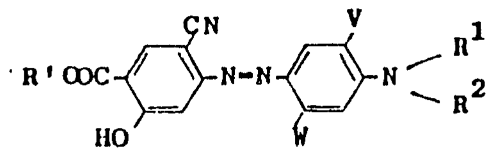

| R' | V | W | $R^1$ | $R^2$ | Farbe auf PES |
|---|---|---|---|---|---|
| (sec.)$C_4H_9$ | H | H | $C_2H_5$ | $C_2H_5$ | scharlach |
| $C_2H_5$ | H | H | $(CH_2)_2OH$ | $(CH_2)_2OH$ | scharlach |
| $CH_3$ | H | H | $(CH_2)_2CN$ | $C_2H_5$ | orange |
| $CH_2-CH=CH_2$ | H | H | $C_6H_5$ | H | orange |
| $CH_3$ | H | H | $(CH_2)_2OCOCH_3$ | $C_2H_5$ | orange |
| $C_2H_5$ | H | H | $(CH_2)_2CN$ | $(CH_2)_2CN$ | goldgelb |
| (i)$C_3H_7$ | H | H | $(CH_2)_2$ OH | $(CH_2)_2OH$ | orange |
| 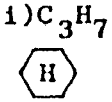 | H | H | $CH_2C_6H_5$ | $CH_3$ | scharlach |
| (i)$C_7H_{15}$ | H | $CH_3$ | $(CH_2)_2OH$ | $(CH_2)_2OH$ | rot |
| $CH_3$ | H | $CH_3$ | $(CH_2)_2OCOCH_3$ | $(CH_2)_2OCOCH_3$ | scharlach |
| $CH_3$ | H | $CH_3$ | $(CH_2)_2CN$ | $(CH_2)_2CN$ | orange |
| $C_2H_5$ | H | $CH_3$ | $CH_2C_6H_5$ | $C_2H_5$ | scharlach |

Tabelle II

Farbstoffe der Formel

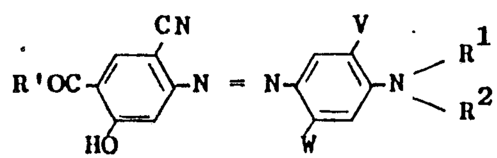

| R' | V | W | $R^1$ | $R^2$ | Farbe auf PES |
|---|---|---|---|---|---|
| $CH_3$ | H | H | $(CH_2)_2OCOCH_3$ | $(CH_2)_2CN$ | orange |
| $CH_3$ | H | H | $(CH_2)_2C_6H_5$ | $(CH_2)_2CN$ | orange |
| $C_2H_5$ | H | H | $C_2H_5$ | $(CH_2)_2OH$ | orange |
| $CH_3$ | H | H | $C_2H_5$ | $(CH_2)_2OCOCH_3$ | orange |
| $C_2H_5$ | H | H | $(CH_2)_2OH$ | $(CH_2)_2CN$ | orange |
| $CH_3$ | H | H | $CH(CH_3)_2COOC_4H_9$ | H | orange |
| $n-C_3-H_7$ | H | H | $CH(C_2H_5)_2$ | H | orange |
| $n-C_4H_9$ | H | H | $(CH_2)_2COOCH_3$ | $CH_2CH-CH_3$ | orange |
| $(CH_2)_2OCH_3$ | H | H | $CH_3O(CH_2)_2$ | $CH_3O(CH_2)_2$ | orange |
| $C_2H_5$ | Cl | H | $(CH_2)_2CN$ | H | orange |
| $CH_2-OCH_3$ | Cl | H | $(CH_2)_2O(CH_2)_2OCH_3$ | H | orange |

Tabelle II (Fortsetzung)

| R' | V | W | $R^1$ | $R^2$ | Farbe auf PES |
|---|---|---|---|---|---|
| $CH_3$ | $CH_3$ | H | $CH(CH_3)_2$ | H | orange |
| $C_6H_5$ | $CH_3$ | H | $(CH_2)_2OC_6H_5$ | H | orange |
| $C_2H_5$ | H | $CH_3$ | $C_2H_5$ | $C_2H_5$ | orange |
| $CH_2-OC_6H_5$ | H | $CH_3$ | $(CH_2)_2OCOCH_3$ | $(CH_2)_2OCOCH_3$ | orange |
| $CH_3$ | H | $CH_3$ | $(CH_2)_2CN$ | $n-C_3H_7$ | orange |
| $i-C_3H_7$ | H | $CH_3$ | $(CH_2)_2CN$ | $(CH_2)_2OH$ | orange |
| $C_2H_5$ | H | $CH_3$ | $n-C_4H_9$ | $(CH_2)_2$ | orange |
| $CH_3$ | H | $CH_3$ | $COCH_2OCH_3$ | H | goldgelb |
| $(CH_2)_2O(CH_2)_2OC_2H_5$ | H | $CH_3$ | $(CH_2)_2Cl$ | $(CH_2)_2Cl$ | orange |
| $CH_3$ | H | $CH_3$ | $(CH_2)_2CN$ | $(CH_2)_2OCOC_2H_5$ | orange |
| $(CH_2)_2CN$ | H | H | $C_6H_5$ | H | orange |
| $C_2H_5$ | $OCH_3$ | H | $(CH_2)_2OCOC_3H_7$ | $(CH_2)_2OCOC_3H_7$ | orange |
| $CH_3$ | $OCH_3$ | H | $CH(CH_3)_2COO(CH_2)_2OCH_3$ | H | orange |
| $(CH_2)_2OH$ | H | $OCH_3$ | $n-C_3H_7$ | $n-C_3H_7$ | orange |

Tabelle II (Fortsetzung)

| R' | V | W | $R^1$ | $R^2$ | Farbe auf PES |
|---|---|---|---|---|---|
| $CH_3$ | $OCH_3$ | $OCH_3$ | $C_2H_5$ | $C_2H_5$ | scharlach |
| $C_2H_5$ | $OCH_3$ | $OCH_3$ | $CH_2CH(OH)CH_2OC_6H_5$ | H | scharlach |
| $CH_3$ | $OCH_3$ | $CH_3$ | $n\text{-}C_4H_9$ | $n\text{-}C_4H_9$ | scharlach |
| $C_2H_5$ | H | $NHCOC_2H_5$ | $CH_2CH(OH)CH_2Cl$ | $CH_2CH(OH)CH_2Cl$ | orange |
| $(i)C_3H_7$ | H | $NHCO\text{-}C_4H_9(n)$ | $C_2H_5$ | $C_2H_5$ | orange |
| $CH_3$ | H | $NHCOC_6H_5$ | $CH_2C_6H_5$ | H | scharlach |
| $[(CH_2)_2O]_2CH_3$ | H | $NHSO_2CH_3$ | $(CH_2)_2OH$ | $(CH_2)_2OH$ | rot |
| $C_2H_5$ | Cl | $NHCOCH_3$ | $CH(C_2H_5)_2$ | H | rot |
| $C_2H_5$ | Cl | $NHCOCH_3$ | $CH_2CH{=}CH_2$ | $CH_2CH{=}CH_2$ | rot |
| $CH_3$ | $CH_3$ | $NHCOCH_3$ | $(CH_2)_2COOCH_3$ | H | rot |
| $C_2H_5$ | $CH_3$ | $NHCOCH_3$ | $(CH_2)_2CN$ | $CH_2CH{=}CH_2$ | |
| $n\text{-}C_4H_9O(CH_2)_2$ | $CH_3$ | $NHCOCH_2OCH_3$ | $C_2H_5$ | $C_2H_5$ | rubin |
| $i\text{-}C_3H_7$ | $OCH_3$ | $NHCOCH_3$ | $(CH_2)_2OCOCH_3$ | $(CH_2)_2OCOCH_3$ | violet |
| $CH_3$ | $OCH_3$ | $NHCOCH_3$ | $(CH_2)_2CN$ | $CH_2CH{=}CH_2$ | violet |
| $C_2H_5$ | $OC_2H_5$ | $NHCOCH_3$ | $(CH_2)_2OH$ | $(CH_2)_2OH$ | violet |

Tabelle II (Fortsetzung)

| R' | V | W | R¹ | R² | Farbe auf PES |
|---|---|---|---|---|---|
| $C_2H_5$ | $OC_2H_5$ | $NHCOCH_3$ | $(CH_2)_2OCOCH_3$ | $(CH_2)_2OCOCH_3$ | violet |
| $CH_3$ | $O(CH_2)_2OCH_3$ | $NHCOC_2H_5$ | $C_2H_5$ | H | violet |
| $CH_3$ | $CH_3$ | $CH_3$ | $(CH_2)_2COOC_2H_5$ | $(CH_2)_2OH$ | scharlach |
| $i\text{-}C_4H_9$ | $OCH_3$ | Cl | $(CH_2)_2C_6H_5$ | $(CH_2)_2CN$ | scharlach |
| $C_2H_5$ | $CH_3$ | $CH_3$ | $(CH_2)_2OH$ | $(CH_2)_2OH$ | scharlach |
| $(i)C_3H_7$ | $OCH_3$ | $CH_3$ | $CH(CH_3)C_2H_5$ | $(CH_2)_2OH$ | orange |
| $CH_3$ | $CH_3$ | Cl | $CH(CH_2OH)C_6H_5$ | H | rot |
| $i\text{-}C_3H_7$ | H | $NHCOCH_3$ | $C_2H_5$ | $(CH_2)_2CN$ | rot |
| $C_2H_5$ | H | $NHCOCH_3$ | $(CH_2)_2CN$ | $CH_2CH{=}CH_2$ | rot |
| $(CH_2)_2OC_6H_5$ | H | $NHCOCH_3$ | $(CH_2)_2CN$ | $(CH_2)_2OH$ | rot |
| $CH_3$ | H | $NHCOC_2H_5$ | $C_2H_5$ | $C_2H_5$ | rot |
| $(i)C_3H_7$ | H | $NHCOC_2H_5$ | $(CH_2)_2CN$ | $(CH_2)_2OCOCH_3$ | rot |
| $C_2H_5$ | H | $NHCOC_2H_5$ | $[(CH_2)_2O]_2H$ | $[(CH_2)_2O]_2H$ | rot |
| $CH_3$ | $O(CH_2)_2OH$ | $NHCOCH_3$ | $CH(CH_3)C_2H_5$ | H | violet |

Tabelle II (Fortsetzung)

Farbstoffe der Formel

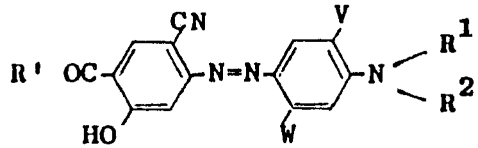

| R' | V | W | $R^1$ | $R^2$ | Farbe auf PES |
|---|---|---|---|---|---|
| (sec.)$C_4H_9$ | H | H | $C_2H_5$ | $C_2H_5$ | scharlach |
| $C_2H_5$ | H | H | $(CH_2)_2OH$ | $(CH_2)_2OH$ | scharlach |
| $CH_3$ | H | H | $(CH_2)_2CN$ | $C_2H_5$ | orange |
| $CH_2-CH=CH_2$ | H | H | $C_6H_5$ | H | orange |
| $CH_3$ | H | H | $(CH_2)_2OCOCH_3$ | $C_2H_5$ | orange |
| $C_2H_5$ | H | H | $(CH_2)_2CN$ | $(CH_2)_2CN$ | goldgelb |
| (i)$C_3H_7$ | H | H | $(CH_2)_2COOC_2H_5$ | $(CH_2)_2OH$ | orange |
| 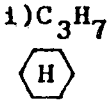 | H | H | $CH_2C_6H_5$ | $CH_3$ | scharlach |
| (i)$C_7H_{15}$ | H | $CH_3$ | $(CH_2)_2OH$ | $(CH_2)_2OH$ | rot |
| $CH_3$ | H | $CH_3$ | $(CH_2)_2OCOCH_3$ | $(CH_2)_2OCOCH_3$ | scharlach |
| $CH_3$ | H | $CH_3$ | $(CH_2)_2CN$ | $(CH_2)_2CN$ | orange |
| $C_2H_5$ | H | $CH_3$ | $CH_2C_6H_5$ | $C_2H_5$ | scharlach |

Tabelle III

Farbstoffe der Formel

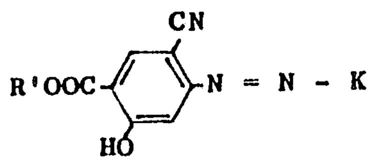

| R' | K | Farbe auf PES |
|---|---|---|
| $C_2H_5$ | 4-Hydroxyphenyl-1 | goldgelb |
| $C_2H_5$ | 4-Hydroxy-3-methylphenyl-1 | goldgelb |
| $C_2H_5$ | 4-N-Phenylaminonaphthyl-1 | rot |
| $CH_3$ | 4-N-(2-Methoxycarbonylethylamino)naphthyl-1 | rot |
| $C_2H_5$ | 2-Hydronaphthyl-1 | rot |
| $H_5C_2O(CH_2)_3$ | 2-Hydroxy-6-N(2-hydroxyethyl)-N-methyl-aminosulfonyl-naphthyl-1 | rot |
| $n-C_3H_7$ | 2.4-Dihydroxychinolinyl-3 | gelb |
| $C_2H_5$ | 3-Hydroxy-5-methyl-N-phenyl-pyrazolyl-4 | gelb |
| $C_2H_5$ | 3-Amino-5-methyl-N-phenyl-pyrazolyl-4 | orange |
| $C_2H_5$ | 2-N-Ethyl-N-(2-hydroxyethyl)amino-thiazolyl-5 | rot |
| $CH_3$ | 2-N-(2-Cyanethyl)-N-(2-acetoxethyl)amino-thiazolyl-5 | scharlach |
| $C_2H_5$ | Carbazolyl-3 | orange |
| $C_2H_5$ | N-Ethylcarbazoylyl-3 | orange |
| $CH_3$ | N-2-Hydroxyethylcarbazolyl-3 | scharlach |

Tabelle IV

Farbstoffe der Formel

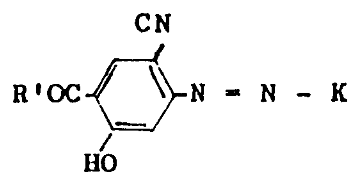

| R' | K | Farbe auf PES |
|---|---|---|
| $CH_3$ | 4-Hydroxyphenyl-1 | goldgelb |
| $(i)C_3H_7$ | 4-N-(2-Acetoxyethylamino)naphthyl-1 | rot |
| $C_2H_5$ | 2-Hydroxynaphthyl-1 | rot |
| $CH_3$ | 2-Hydroxy-6-N(2-hydroxyethyl)-N-methyl-aminosulfonyl-naphthyl-1 | rot |
| $CH_3$ | 3-Hydroxy-5-methyl-N-phenyl-pyrazolyl-4 | gelb |
| $C_2H_5$ | 2-N-Ethyl-N-(2-hydroxyethyl)amino-thiazolyl-5 | rot |
| $CH_3$ | 2-N-(2-Cyanethyl)-N-(2-acetoxethyl)amino-thiazolyl-5 | scharlach |
| $(n)C_3H_7$ | Carbazolyl-3 | orange |
| $C_2H_5$ | N-Ethylcarbazolyl-3 | orange |

Tabelle V

Farbstoffe der Formel

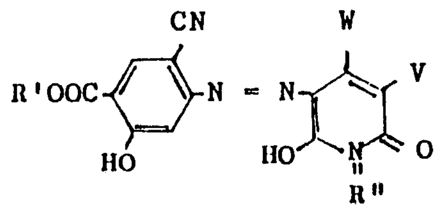

| R' | R'' | V | W | Farbe auf PES |
|---|---|---|---|---|
| $C_2H_5$ | H | CN | $CH_3$ | gelb |
| $(i)C_3H_7$ | H | CN | $CH_3$ | gelb |
| $C_2H_5$ | H | $CONH_2$ | $CH_3$ | gelb |
| $(i)C_8H_{17}$ | H | CN | $C_6H_5$ | gelb |
| $(n)C_5H_{11}$ | $CH_3$ | CN | $CH_3$ | gelb |
| H | $CH_3$ | CN | $CH_3$ | gelb |
| $CH_3O(CH_2)_2$ | $C_2H_5$ | CN | $CH_3$ | gelb |
| $CH_3$ | $C_2H_5$ | $NO_2$ | $CH_3$ | gelb |
| $n-C_4H_9$ | $n-C_4H_9$ | CN | $CH_3$ | gelb |
| $C_2H_5$ | $n-C_8H_{17}$ | CN | $CH_3$ | gelb |

Tabelle VI

Farbstoffe der Formel

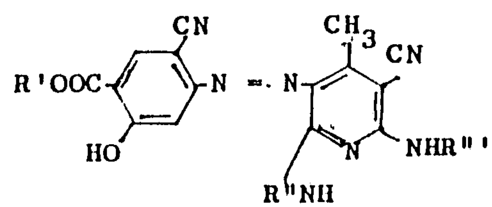

| R' | R'' | R''' | Farbe auf PES |
|---|---|---|---|
| $CH_3$ | $CH_3O(CH_2)_3$ | $CH_3O(CH_2)_3$ | scharlach |
| $CH_2-OCH_3$ | H | $(CH_2)_3O(CH_2)_2OC_6H_5$ | scharlach |
| $C_2H_5$ | H | $(CH_2)_3O(CH_2)_4OH$ | scharlach |
| $CH_2-O(CH_2)_2-OCH_3$ | H | $(CH_2)_3O(CH_2)_4OH$ | rot |
| $C_2H_5$ | H | $(CH_2)_3O(CH_2)_4OH$ | violett |
| $(n)C_4H_9$ | $CH_3O(CH_2)_3$ | $CH_3O(CH_2)_3$ | scharlach |

Tabelle VII

Farbstoffe der Formel

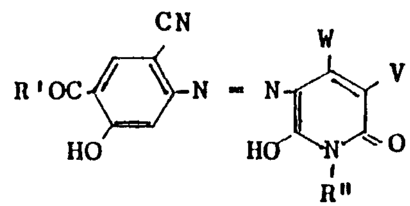

| R' | R'' | V | W | Farbe auf PES |
|---|---|---|---|---|
| $CH_3$ | H | CN | $CH_3$ | gelb |
| $(i)C_3H_7$ | H | CN | $CH_3$ | gelb |
| $C_2H_5$ | H | $CONH_2$ | $CH_3$ | gelb |
| $(i)C_8H_{17}$ | H | CN | $C_6H_5$ | gelb |
| $(n)C_5H_{11}$ | $CH_3$ | CN | $CH_3$ | gelb |
| $CH_3O(CH_2)_2$ | $C_2H_5$ | CN | $CH_3$ | gelb |
| $CH_3$ | $C_2H_5$ | $NO_2$ | $CH_3$ | gelb |
| $n-C_4H_9$ | $n-C_4H_9$ | CN | $CH_3$ | gelb |
| $C_2H_5$ | $n-C_8H_{17}$ | CN | $CH_3$ | gelb |

Tabelle VIII

Farbstoffe der Formel

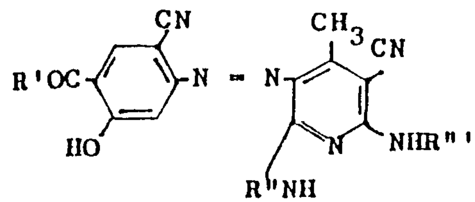

| R' | R'' | R''' | Farbe auf PES |
|---|---|---|---|
| $CH_3$ | $CH_3O(CH_2)_3$ | $CH_3O(CH_2)_3$ | scharlach |
| $CH_2-OCH_3$ | H | $(CH_2)_3O(CH_2)_2OC_6H_5$ | scharlach |
| $C_2H_5$ | H | $(CH_2)_3O(CH_2)_4OH$ | scharlach |
| $CH_2-O(CH_2)_2-OCH_3$ | H | $(CH_2)_3O(CH_2)_4OH$ | rot |
| $C_2H_5$ | H | $(CH_2)_3O(CH_2)_4OH$ | violett |
| $(n)C_4H_9$ | $CH_3O(CH_2)_3$ | $CH_3O(CH_2)_3$ | scharlach |

**Patentansprüche**

1. Verfahren zur Herstellung von weißen oder verschiedenfarbigen Mustern auf farbigem Untergrund auf Textilmaterialien, enthaltend oder bestehend aus hydrophoben synthetischen Fasern, insbesondere Polyesterfasern, oder auf Textilmaterialien auf Basis von Mischfasern aus Polyester und Cellulose, insbesondere Polyester-Baumwollfasern, durch Imprägnieren der Materialien mit Farbflotten, die neben den üblichen Färbe- und Klotzhilfsmitteln weißätzbare Dispersionsfarbstoffe, und, sofern Mischfasertextilien verarbeitet werden, gewünschtenfalls weißätzbare Reaktivfarbstoffe, die als Reaktivanker eine Gruppe der Formel

$-SO_2-CH_2-CH_2-hal$
$-SO_2-CH_2-CH_2-O-SO_3M$
$-NH-SO_2-CH_2-CH_2-O-SO_3M$
$-SO_2-CH=CH_2$

aufweisen, enthalten und Aufdrucken einer Ätzreservepaste, die gewünschtenfalls neben dem Atzreservemittel noch ätzmittelbeständige Farbstoffe enthält, in dem gewünschten Muster, wobei die Reihenfolge von Imprägnieren und Aufdrucken der Ätzreservepaste beliebig ist und das Imprägnieren auch durch Bedrucken mit einer entsprechenden Druckpaste ersetzt werden kann und anschließende Wärmebehandlung bei Temperaturen von 100 bis 230 °C, wobei für den Fall, daß keine weißätzbaren Reaktivfarbstoffe anwesend sind als Ätzreservemittel eine Base, die in 5 %iger wäßriger Lösung mindestens einen pH-Wert von 8 hervorbringt oder ein Ätzreservemittel auf Sulfitbasis, das ein Alkalisulfit oder aber ein Alkalihydrogensulfit in Kombination mit Alkalicarbonat oder Alkalihydrogencarbonat und gegebenenfalls einem Aldehyd und gegebenenfalls ein nichtionogenes Detergens enthält, für den Fall, daß die obenbezeichneten weißätzbaren Reaktivfarbstoffe vorhanden sind, das Ätzreservemittel auf Sulfitbasis eingesetzt wird, dadurch gekennzeichnet, daß als ätzbare Dispersionsfarbstoffe solche der Formel I

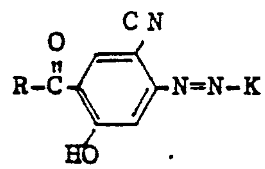

(I)

in der R gegebenenfalls substituiertes Alkyl mit 1 bis 8 C-Atomen, gegebenenfalls substituiertes Alkoxy mit 1 bis 8 C-Atomen und K den Rest einer Kupplungskomponente der Benzol-, Naphthalin-, Pyridin-, Benzpyridin-, Diazol-, Thiazol-, Indol-, Carbazol-, Oxazol-, Diazin-Reihe oder aus der Reihe der enolisierbaren 1,3-Dicarbonylverbindungen bedeuten und die in ihrem Molekül höchstens eine veresterte Carboxylgruppe aufweisen, eingesetzt werden.

2. Verfahren gemäß Anspruch 1, dadurch gekennzeichnet, daß ätzbare Dispersionsfarbstoffe der Formel I eingesetzt werden, worin R gegebenenfalls substituiertes Alkyl mit 1 bis 4 C-Atomen oder gegebenenfalls substituiertes Alkoxy mit 1 bis 4 C-Atomen bedeutet.

3. Verfahren gemäß den Ansprüchen 1 oder 2, dadurch gekennzeichnet, daß ätzbare Dispersionsfarbstoffe der Formel I eingesetzt werden, worin die für R stehenden Alkyl- bzw. Alkoxyreste unsubstituiert oder gegebenenfalls unabhängig voneinander ein- oder zweifach substituiert sind, durch Chlor, Brom, Cyan, Hydroxy, Alkoxy mit 1 bis 4 C-Atomen, Alkenoxy mit 3 oder 4 C-Atomen, Hydroxyalkoxy mit 2 bis 4 C-Atomen, Alkoxyalkoxy mit insgesamt 3 bis 8 C-Atomen, Alkylcarbonyloxy mit 2 bis 4 C-Atomen, gegebenenfalls substituiertes Benzoyloxy, gegebenenfalls substituiertes Phenyl, gegebenenfalls substituiertes Phenoxy und als dritten Substituenten eine OH-Gruppe tragen können.

4. Verfahren gemäß den Ansprüchen 1 bis 3, dadurch gekennzeichnet, daß ätzbare Dispersionsfarbstoffe der Formel I eingesetzt werden, worin K der Rest einer Kupplungskomponente der Anilin-, Naphthol-2, Naphthylamin-1-, 6-Hydroxypyridon-2-, Aminopyridin-, 2-Aminothiazol-, Indol- oder Carbazol-Reihe ist.

5. Verfahren gemäß den Ansprüchen 1 bis 4, dadurch gekennzeichnet, daß ätzbare Dispersionsfarbstoffe der Formel I eingesetzt werden, worin K der Rest eines Anilinderivats der Formel

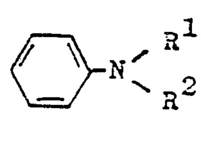

ist, worin $R^1$ Wasserstoff, Alkyl, Alkenyl und $R^2$ Alkyl, Alkenyl, Cycloalkyl, Phenyl, Alkylcarbonyl, Benzoyl, Alkylsulfonyl oder Phenylsulfonyl bedeutet, deren N-Alkyl-, N-Alkenyl-, N-Cycloalkyl-, N-Phenyl-, N-Acyl-, N-Alkylsulfonyl- und N-Phenylsulfonylrest gegebenenfalls substituiert sein kann und deren Kern ggf. durch gegebenenfalls substituiertes Alkyl mit 1 bis 4 C-Atomen, gegebenenfalls substituiertes Alkoxy mit

1 bis 4 C-Atomen, Alkenoxy mit 3 oder 4 C-Atomen, gegebenenfalls substituiertes Phenoxy, gegebenenfalls substituiertes Phenyl, Fluor, Chlor, Brom, gegebenenfalls substituiertes Alkylcarbonylamino, gegebenenfalls substituiertes Benzoylamino, Alkenylcarbonylamino, gegebenenfalls substituiertes Alkylsulfonylamino, gegebenenfalls substituiertes Phenylsulfonylamino, gegebenenfalls substituiertes N-Alkyl-N-alkylsulfonyl- oder N-Alkyl-N-phenylsulfonylamino, gegebenenfalls substituiertes Alkoxy-, Alkenoxy-, Cycloalkoxy- oder Phenoxycarbonylamino, gegebenenfalls substituiertes Alkyl-, alkenyl-, Cycloalkyl- oder Phenylaminocarbonylamino oder Hydroxy ein- oder mehrfach substituiert sein kann oder eines Phenols, dessen Kern durch Alkyl mit 1 bis 4 C-Atomen oder gegebenenfalls substituiertes Alkylcarbonyl- oder Benzoylamino substituiert sein kann.

6. Verfahren gemäß den Ansprüchen 1 bis 3, dadurch gekennzeichnet, daß ätzbare Dispersionsfarbstoffe der Formel I eingesetzt werden, worin K der Rest eines 1- oder 2-Naphthylamins der Formel

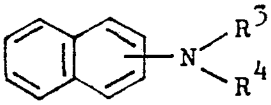

ist, worin $R^3$ Wasserstoff, Alkyl, Alkenyl und $R^4$ Alkyl, Alkenyl, Cycloalkyl, Phenyl, Alkylcarbonyl, Benzoyl, Alkylsulfonyl oder Phenylsulfonyl bedeutet, deren N-Alkyl- oder N-Phenyl-Reste gegebenenfalls substituiert sein können und deren Kern gegebenenfalls substituiert sein kann durch Alkyl mit 1 bis 4 C-Atomen, Alkoxy mit 1 bis 4 C-Atomen, Chlor, Brom, Aminosulfonyl, N-mono- oder N,N-di-alkylsubstituiertes Aminosulfonyl, wobei die Alkylreste ihrerseits unabhängig voneinander gegebenenfalls ein- oder mehrfach substituiert sein können, oder eines 1- oder 2-Naphthols, dessen Kern gegebenenfalls substituiert sein kann durch Aminocarbonyl, gegebenenfalls unabhängig voneinander substituiertes Mono- oder Dialkyl- oder Phenylaminocarbonyl, gegebenenfalls substituiertes Alkoxy-, Alkenoxy-, Cycloalkoxy- oder Phenoxycarbonyl, Amino, gegebenenfalls substituiertes Alkylcarbonylamino, Alkenylcarbonylamino, gegebenenfalls substituiertes Benzoylamino, gegebenenfalls substituiertes Alkylsulfonylamino, gegebenenfalls substituiertes Phenylsulfonylamino, Alkyl mit 1 bis 4 C-Atomen, Alkoxy mit 1 bis 4 C-Atomen, Chlor oder Brom, Aminosulfonyl, Mono- und Di-alkylaminosulfonyl, deren Alkylreste gegebenenfalls unabhängig voneinander substituiert sein können.

7. Verfahren gemäß den Ansprüchen 1 bis 3, dadurch gekennzeichnet, daß ätzbare Dispersionsfarbstoffe der Formel I eingesetzt werden, worin K der Rest eines 6-Hydroxypyridons-2- der Formel II

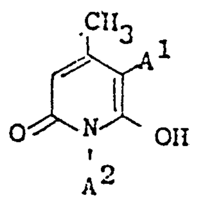

(II)

in der $A^1$ Cyan, Nitro, gegebenenfalls substituiertes Aminocarbonyl, Wasserstoff und $A^2$ Wasserstoff, gegebenenfalls substituiertes Amino oder gegebenenfalls substituiertes Alkyl mit 1 bis 8 C-Atomen bedeuten oder eines Aminopyridinderivats der Formel III

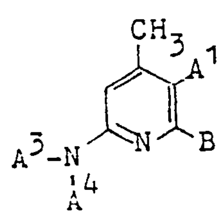

(III)

in der $A^3$ und $A^4$ Wasserstoff oder unabhängig voneinander gegebenenfalls substituiertes Alkyl mit 1 bis 8 C-Atomen, Alkenyl mit 3 bis 5 C-Atomen, Cycloalkyl mit 5 oder 6 C-Atomen und B für $NA^5A^6$ oder für gegebenenfalls substituiertes Alkoxy oder Alkylthio mit 1 bis 8 C-Atomen stehen und $A^5$ und $A^6$ unabhängig voneinander Wasserstoff oder gegebenenfalls substituiertes Alkyl mit 1 bis 8 C-Atomen, Alkenyl mit 3 bis 5 C-Atomen, Cycloalkyl mit 5 oder 6 C-Atomen bedeuten, und $A^1$ die oben genannten Bedeutungen hat, oder eines Aminopyridinderivats der Formel IV ist,

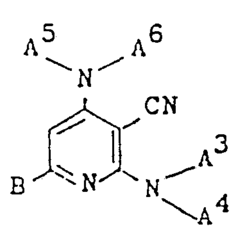

(IV)

in der $A^3$ bis $A^6$ und B die oben genannten Bedeutungen haben.

8. Verfahren gemäß den Ansprüchen 1 bis 5, dadurch gekennzeichnet, daß ätzbare Dispersionsfarbstoffe der Formel I eingesetzt werden, worin die in $R^1$, $R^2$ und $A^3$ bis $A^6$ der Kupplungskomponenten K enthaltenen Alkylreste 1 bis 8 C-Atome, Alkenylreste 3 bis 5 C-Atome, Cycloalkylreste 5 oder 6 C-Atome aufweisen und Arylreste Phenyl- oder Naphthylreste sind.

9. Verfahren gemäß den Ansprüchen 1 bis 6, dadurch gekennzeichnet, daß ätzbare Dispersionsfarbstoffe der Formel I eingesetzt werden, worin die Alkylreste der Kupplungskomponenten unsubstituiert oder durch die Substituenten Hydroxy ; Alkoxy mit 1 bis 4 C-Atomen ; Alkenyloxy mit 3 bis 5 C-Atomen ; gegebenenfalls substituiertes Phenoxy ; Cycloalkoxy mit 5 oder 6 C-Atomen ; Hydroxyalkoxy ; Alkylcarbonyloxyalkoxy oder Phenoxyalkoxy mit 2 bis 4 C-Atomen im Alkoxy- bzw. im Alkylcarbonylrest ; Alkoxyalkoxy mit 3 bis 8 C-Atomen ; Hydroxyalkoxyalkoxy, Alkylcarbonyloxyalkoxyalkoxy oder phenoxyalkoxyalkoxy mit 4 bis 12 C-Atomen im Alkoxyalkoxyrest und 2 bis 4 C-Atomen im Alkylcarbonylrest ; Alkoxyalkoxyalkoxy mit 5 bis 16 C-Atomen ; gegebenenfalls durch Hydroxy, Chlor, Brom, Alkoxy mit 1 bis 4 C-Atomen oder Phenoxy substituiertes Alkylcarbonyloxy mit 2 bis 4 C-Atomen ; gegebenenfalls substituiertes Benzoyloxy ; gegebenenfalls durch Hydroxy, Chlor, Brom, Alkoxy mit 1 bis 4 C-Atomen oder Phenoxy substituiertes Alkoxycarbonyloxy mit 1 bis 4 C-Atomen in der Alkoxygruppe ; Alkenoxycarbonyloxy mit 3 bis 5 C-Atomen in der Alkenoxygruppe ; Cycloalkoxycarbonyloxy mit 5 oder 6 C-Atomen in der Cycloalkoxygruppe ; gegebenenfalls substituiertes Phenoxycarbonyloxy ; gegebenenfalls unabhängig voneinander durch Hydroxy, Chlor, Brom, Alkoxy mit 1 bis 4 C-Atomen oder Phenoxy substituiertes Monoalkylaminocarbonyloxy mit 1 bis 8 C-Atomen in der Alkylaminogruppe ; gegebenenfalls substituiertes Monoarylaminocarbonyloxy ; gegebenenfalls substituiertes Phenyl, Chlor ; Brom ; Cyan ; und für den Fall, daß R gegebenenfalls substituiertes Alkyl ist, Alkoxycarbonyl mit 1 bis 8 C-Atomen in der gegebenenfalls substituierten Alkoxygruppe ; Alkenoxycarbonyl mit 3 bis 5 C-Atomen in der Alkenoxygruppe ; Cycloalkoxycarbonyl mit 5 oder 6 C-Atomen in der Cycloalkoxygruppe ; Phenoxycarbonyl mit ggf. substituiertem Phenoxyrest substituiert sein können und die Naphthyl- oder Phenylreste durch Fluor, Chlor, Brom, Trifluormethyl Alkyl mit 1 bis 4 C-Atomen, Alkoxy mit 1 bis 4 C-Atomen mono- oder disubstituiert sein können.

10. Die nach dem Verfahren der Ansprüche 1 bis 9 colorierten Textilmaterialien.

## Claims

1. Process for the preparation of white patterns or patterns of various colours on a coloured substrate on textile materials comprising or consisting of hydrophobic synthetic fibres, in particular polyester fibres, or on textile materials based on mixed fibres composed of polyester and cellulose, in particular polyester-cotton fibres, by impregnating the materials with dye liquors which, besides the customary dyeing and padding auxiliaries, contain disperse dyestuffs which are dischargeable to white, and if mixed fibre textiles are processed, contain, if desired, reactive dyestuffs which are dischargeable to white and which have, as a reactive anchor, a group of the formula

$-SO_2-CH_2CH_2-hal,$
$-SO_2-CH_2CH_2-O-SO_3M$
$-NH-SO_2-CH_2CH_2-O-SO_3M$ and
$-SO_2-CH=CH_2$

and printing on, in the desired pattern, a discharge reserve paste which, besides the discharge reserving agent, also contains, if desired, dyestuffs which are resistant to discharging agents, in which connection the sequence of impregnating and printing on the discharge reserve paste is immaterial and it is also possible to replace the impregnation by printing with an appropriate printing paste, and by subsequently subjecting the material to a heat treatment at temperatures of 100 to 230 °C, in which process, if no reactive dyestuffs which are dischargeable to white are present, the discharge reserving agent employed is a base which produces a pH value of at least 8 in a 5 % strength aqueous solution, or is a discharge reserving agent based on sulphites which contains an alkali metal sulphite or an alkali metal bisulphite in combination with an alkali metal carbonate or bicarbonate and, if appropriate, an aldehyd, and which, optionally contains a nonionic detergent, and if the abovementioned reactive dyestuffs which are dischargeable to white are present, the discharge reserving agent based on sulphites is employed, characterised in that the dischargeable disperse dyestuffs employed are dyestuffs having the formula I

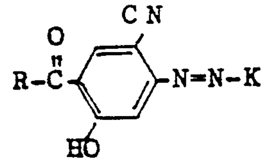

(I)

in which R is optionally substituted alkyl having 1 to 8 carbon atoms, optionally substituted alkoxy having 1 to 8 C atoms, and K is the radical of a coupling component of the benzene, naphthalene, pyridine, benzpyridine, diazole, thiazole, indole, carbazole, oxazole or diazine series or the series of enolisable 1,3-dicarbonyl compounds, the molecule of which dyestuffs contains not more than one esterified carboxyl group.

2. Process of Claim 1, characterised in that dischargeable disperse dyestuffs having the formula I are employed wherein R is alkyl having 1 to 4 C atoms, substituted alkyl having 1 to 4 C atoms, alkoxy having 1 to 4 C atoms and substituted alkoxy having 1 to 4 C atoms.

3. Process of Claim 1 or 2, characterised in that dischargeable disperse dyestuffs of the formula I are employed wherein the alkyl or alkoxy radicals represented by R are unsubstituted or optionally monosubstituted or disubstituted by a substituent selected from the group consisting of chlorine, bromine, cyano, hydroxyl, alkoxy having 1 to 4 C atoms, alkeneoxy having 3 or 4 C atoms, hydroxyalkoxy having 2 to 4 C atoms, alkoxyalkoxy having a total of 3 to 8 C atoms, alkylcarbonyloxy having 2 to 4 C atoms, benzoyloxy, substituted benzoyloxy, phenyl, substituted phenyl, phenoxy, substituted phenoxy ; and carrying an OH group as third substituent.

4. Process of Claims 1 to 3, characterised in that dischargeable disperse dyestuffs of the formula I are employed wherein K is the radical of a coupling component of the aniline, 2-naphthol, 1-naphthylamine, 6-hydroxypyrid-2-one, aminopyridine, 2-aminothiazole, indole or carbazole series.

5. Process of Claims 1 to 4, characterised in that dischargeable disperse dyestuffs of the formula I are employed wherein K is the radical of an aniline derivative of the formula

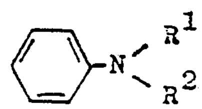

wherein $R^1$ is hydrogen, alkyl or alkenyl and $R^2$ is alkyl, alkenyl, cycloalkyl, phenyl, alkylcarbonyl, benzoyl, alkylsulphonyl or phenylsulphonyl, and in which the N-alkyl, N-alkenyl, N-cycloalkyl, N-phenyl, N-acyl, N-alkylsulphonyl and N-phenylsulphonyl radical are unsubstituted or substituted and in which the nucleus is unsubstituted or monosubstituted or polysubstituted by one or several substituents selected from the group consisting of alkyl having 1 to 4 C atoms, substituted alkyl having 1 to 4 C atoms, alkoxy having 1 to 4 C atoms, substituted alkoxy having 1 to 4 C atoms, alkeneoxy having 3 or 4 C atoms, phenoxy, substituted phenoxy, phenyl, substituted phenyl, fluorine, chlorine, bromine, alkylcarbonylamino, substituted alkylcarbonylamino, benzoylamino, substituted benzoylamino, alkenylcarbonylamino, alkylsulphonylamino, substituted alkylsulphonylamino, phenylsulphonylamino, substituted phenylsulphonylamino, N-alkyl-N-alkylsulphonylamino or N-alkyl-N-phenylsulphonylamino, substituted N-alkyl-N-alkylsulphonylamino or N-alkyl-N-phenylsulphonylamino, alkoxycarbonylamino, substituted alkoxycarbonylamino, alkeneoxycarbonylamino, cycloalkoxycarbonylamino or phenoxycarbonylamino, alkylaminocarbonylamino, substituted alkylaminocarbonylamino, alkenylaminocarbonylamino, cycloalkylaminocarbonylamino, phenylaminocarbonylamino and hydroxyl, or is a phenol in which the nucleus is unsubstituted or substituted by alkyl having 1 to 4 C atoms, alkylcarbonylamino or benzoylamino, substituted alkylcarbonylamino or benzoylamino.

6. Process of Claims 1 to 3, characterised in that K is the radical of a 1-naphthylamine of 2-naphthylamine of the formula

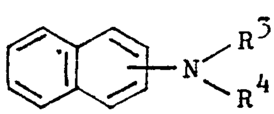

wherein $R^3$ is hydrogen, alkyl and alkenyl and $R^4$ is alkyl, alkenyl, cycloalkyl, phenyl, alkylcarbonyl, benzoyl, alkylsulphonyl or phenylsulphonyl, in which the N-alkyl or N-phenyl radicals are unsubstituted or substituted and in which the nucleus is unsubstituted or substituted by a substituent selected from the group consisting of alkyl having 1 to 4 C atoms, alkoxy having 1 to 4 C atoms, chlorine, bromine, aminosulphonyl, N-monoalkyl-substituted aminosulphonyl or N,N-dialkylsubstituted aminosulphonyl, the alkyl radicals in turn, independently of one another, being unsubstituted, monosubstituted or polysubstituted ; or of a 1-naphthol or 2-naphthol in which the nucleus is unsubstituted or substituted by aminocarbonyl, monoalkylaminocarbonyl, dialkylaminocarbonyl or phenylaminocarbonyl, the alkyl and phenyl groups of which are, independently of one another, unsubstituted or substituted, alkoxycarbonyl, substituted alkoxycarbonyl, alkeneoxycarbonyl, cycloalkoxycarbonyl or phenoxycarbonyl, amino, alkylcarbonylamino, substituted alkylcarbonylamino, alkenylcarbonylamino, benzoylamino, substituted benzoylamino, alkylsulphonylamino, substituted benzoylamino, alkylsulphonylamino, substituted alkylsulphonylamino, phenylsulphonylamino, substituted phenylsulphonylamino, alkyl having 1 to 4 C atoms, alkoxy having 1 to 4 C atoms, chlorine or bromine, aminosulphonyl and monoalkylaminosulphonyl and dialkylaminosulphonyl in which the alkyl radicals, independently of one another, are unsubstituted or substituted.

7. Process of Claims 1 to 3, characterised in that dischargeable disperse dyestuffs of the formula I are employed, wherein K is the radical of a 6-hydroxypyrid-2-one of the formula II

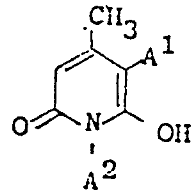

(II)

in which $A^1$ is cyano, nitro, aminocarbonyl, substituted aminocarbonyl or hydrogen and $A^2$ is hydrogen, amino, substituted amino, or alkyl having 1 to 8 C atoms, substituted alkyl having 1 to 8 C atoms, or K is a radical of an amino pyridine derivative of the formula III

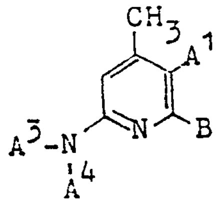

(III)

in which $A^3$ and $A^4$ are hydrogen or, independently of one another, are alkyl having 1 to 8 C atoms, substituted alkyl having 1 to 8 C atoms, alkenyl having 3 to 5 C atoms or cycloalkyl having 5 or 6 C atoms and B is $NA^5A^6$ or alkoxy or alkylthio having 1 to 8 C atoms or substituted alkoxy or alkylthio having 1 to 8 C atoms and $A^5$ and $A^6$ independently of one another are hydrogen, or alkyl having 1 to 8 C atoms, substituted alkyl having 1 to 8 C atoms, alkenyl having 3 to 5 C atoms or cycloalkyl having 5 or 6 C atoms and A has the meanings mentioned above, or K is a radical of an aminopyridine derivative of the formula IV

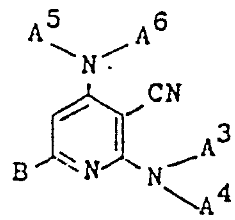

(IV)

in which $A^3$ to $A^6$ and B have the meanings mentioned above.

8. Process of Claims 1 to 5, characterised in that dischargeable disperse dyestuffs of the formula I are employed wherein the alkyl radicals present in $R^1$, $R^2$ and $A^3$ to $A^6$ of the coupling components K contain 1 to 8 C atoms, the alkenyl radicals contain 3 to 5 C atoms and the cycloalkyl radicals contain 5 or 6 C atoms and the aryl radicals are phenyl or naphthyl radicals.

9. Process of Claims 1 to 6, characterised in that dischargeable disperse dyestuffs of the formula I are employed wherein the alkyl radicals of the coupling components are unsubstituted or substituted by a substituent selected from the group consisting of hydroxy, alkoxy having 1 to 4 C atoms, alkenyloxy having 3 to 5 C atoms, phenoxy, substituted phenoxy, cycloalkoxy having 5 or 6 C atoms, hydroxyalkoxy, alkylcarbonyloxyalkoxy or phenoxyalkoxy having 2 to 4 C atoms in the alkoxy radical or in the alkylcarbonyl radical, alkoxyalkoxy having 3 to 8 C atoms, hydroxyalkoxyalkoxy, alkylcarbonyloxyalkoxyalkoxy or phenoxyalkoxyalkoxy having 4 to 12 C atoms in the alkoxyalkoxy radical and 2 to 4 C atoms in the alkylcarbonyl radical ; alkoxyalkoxyalkoxy having 5 to 16 C atoms ; alkylcarbonyloxy having 2 to 4 C atoms ; alkylcarbonyloxy having 2 to 4 C atoms substituted by hydroxyl, chlorine, bromine, alkoxy having 1 to 4 C atoms or phenoxy and benzoyloxy and substituted benzoyloxy ; alkoxycarbonyloxy having 1 to 4 C atoms in the alkoxy group, alkoxycarbonyloxy having 1 to 4 C atoms in the alkoxy group substituted by hydroxy, chlorine, bromine, alkoxy having 1 to 4 C atoms, or phenoxy ; alkeneoxycarbonyloxy having 3 to 5 C atoms in the alkeneoxy group ; cycloalkoxycarbonyloxy having 5 or 6 C atoms in the cycloalkoxy group ; phenoxycarbonyloxy, substituted phenoxycarbonyloxy ; monoalkylaminocarbonyloxy unsubstituted or substituted, by hydroxyl, chlorine, bromine, alkoxy having 1 to 4 C atoms or phenoxy and which has 1 to 8 C atoms in the alkylamino group ; monoarylaminocarbonyloxy, substituted monoarylaminocarbonyloxy ; phenyl, substituted phenyl ; chlorine ; bromine ; and cyano ; and in the event that R is alkyl or substituted alkyl, alkoxycarbonyl having 1 to 8 C atoms in the alkoxy group, which is unsubstituted or substituted ; alkeneoxycarbonyl having 3 to 5 C atoms in the alkeneoxy group ; cycloalkoxycarbonyl having 5 or 6 C atoms in the cycloalkoxy group ; and phenoxycarbonyl in which the phenoxy radical is

unsubstituted or substituted, and the naphthyl or phenyl radicals are monosubstituted or disubstituted by fluorine, chlorine, bromine, trifluoromethyl, alkyl having 1 to 4 C atoms or alkoxy having 1 to 4 C atoms.

10. The textile material which has been coloured by the process of any one of Claims 1 to 9.

**Revendications**

1. Procédé de réalisation de dessins blancs ou diversement colorés sur un fond coloré sur des matières textiles contenant ou constituées de fibres synthétiques hydrophobes, en particulier de fibres de polyester, ou sur des matières textiles à base de fibres mélangées de polyester et de cellulose, en particulier des fibres de polyester-coton, par imprégnation des matières avec des bains de teinture qui contiennent, outre les adjuvants de teinture et de foulardage habituels, des colorants en dispersion rongeables en blanc, et, si l'on met en œuvre des textiles de fibres mélangées, le cas échéant des colorants réactifs rongeables en blanc, qui présentent comme ancrage réactif un groupe répondant aux formules :

$-SO_2-CH_2-CH_2-hal$
$-SO_2-CH_2-CH_2-O-SO_3M$
$-NH-SO_2-CH_2-CH_2-O-SO_3M$
$-SO_2-CH=CH_2$

et impression d'une pâte de réserve rongeante, qui contient, si on le désire, outre l'agent de réserve rongeante, des colorants résistant aux rongeants, dans le dessin souhaité, l'ordre des opérations d'imprégnation et d'impression de la pâte de réserve rongeante étant quelconque, et l'imprégnation pouvant aussi être remplacée par une impression avec une pâte d'impression appropriée, puis un traitement thermique à des températures de 100 à 230 °C, en utilisant comme agents de réserve rongeants, dans le cas où aucun colorant réactif rongeable en blanc n'est présent, une base qui produit en solution aqueuse à 5 % un pH d'au moins 8 ou un agent de réserve rongeante à base de sulfite qui contient un sulfite alcalin ou encore un bisulfite alcalin associé à un carbonate alcalin ou à un bicarbonate alcalin, et le cas échéant à un aldéhyde, et le cas échéant un détergent non ionique, et dans le cas où les colorants réactifs rongeables en blanc indiqués ci-dessus sont présents, l'agent de réserve à base de sulfite caractérisé en ce qu'on utilise comme colorants en dispersion rongeables, des colorants répondant à la formule I

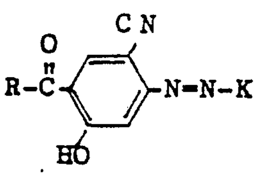

(I)

dans laquelle R désigne un alkyle en $C_1$-$C_8$ éventuellement substitué, un alcoxy en $C_1$-$C_8$ éventuellement substitué, et K est le radical d'un constituant de copulation de la série du benzène, du naphtalène, de la pyridine, de la benzopyridine, du diazol, du thiazole, de l'indole, du carbazole, de l'oxazole, de la diazine ou encore de la série des composés 1,3-dicarbonylés énolisables et qui contiennent dans leur molécule un groupe carboxyle estérifié au maximum.

2. Procédé selon la revendication 1, caractérisé en ce qu'on utilise des colorants en dispersion rongeables répondant à la formule I, dans laquelle R désigne un alkyle en $C_1$-$C_4$ éventuellement substitué ou un alcoxy en $C_1$-$C_4$ éventuellement substitué.

3. Procédé selon l'une quelconque des revendications 1 ou 2, caractérisé en ce qu'on utilise des colorants en dispersion rongeables répondant à la formule I, dans laquelle les radicaux alkyle ou alcoxy représentés par R sont non substitués ou éventuellement mono- ou di-substitués indépendamment les uns des autres, par un chlore, un brome, un cyano, un hydroxy, un alcoxy en $C_1$-$C_4$, un alcénoxy en $C_3$ ou $C_4$, un hydroxyalcoxy en $C_2$-$C_4$, un alcoxyalcoxy ayant en tout 3 à 8 atomes de carbone, un alkylcarbonyloxy en $C_2$-$C_4$, un benzoyloxy éventuellement substitué, un phényle éventuellement substitué, un phénoxy éventuellement substitué, et peuvent porter comme troisième substituant, un groupe OH.

4. Procédé selon les revendications 1 à 3, caractérisé en ce qu'on utilise des colorants en dispersion rongeables répondant à la formule I, dans laquelle K est le radical d'un constituant de copulation de la série de l'aniline, du naphthol-2, de la naphtylamine-1, de la 6-hydroxypyridone-2, de l'aminopyridine, du 2-aminothiazole, de l'indole ou du carbazole.

5. Procédé selon les revendications 1 à 4, caractérisé en ce qu'on utilise des colorants en dispersion rongeables répondant à la formule I, dans laquelle K est le radical d'un dérivé de l'aniline répondant à la formule

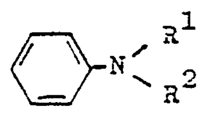

dans laquelle $R^1$ représente l'hydrogène, un alkyle, un alcényle, et $R^2$ un alkyle, un alcényle, un cycloalkyle, un phényle, un alkylcarbonyle, un benzoyle, un alkylsulfonyle ou un phénylsulfonyle, dont le radical N-alkyle, N-alcényle, N-cycloalkyle, N-phényle, N-acyle, N-alkylsulfonyle et N-phénylsulfonyle peut le cas échéant être substitué et dont le noyau peut le cas échéant être mono- ou poly-substitué par un alkyle en $C_1$-$C_4$ éventuellement substitué, un alcoxy en $C_1$-$C_4$ éventuellement substitué, un alcénoxy en $C_3$ ou $C_4$, un phénoxy éventuellement substitué, un phényle éventuellement substitué, le fluor, le chlore, le brome, un alkylcarbonylamino éventuellement substitué, un benzoylamino éventuellement substitué, un alcénylcarbonylamino, un alkylsulfonylamino éventuellement substitué, un phénylsulfonylamino éventuellement substitué, un N-alkyl-N-alkyl-sulfonyl- ou N-alkyl-N-phénylsulfonylamino éventuellement substitués, un alcoxy, un alcénoxy, un cycloalcoxy ou un phénoxycarbonylamino éventuellement substitués, un alkyle, un alcényle, un cycloalkyle- ou un phénylaminocarbonylamino éventuellement substitués ou un hydroxy, ou d'un phénol dont le noyau peut être substitué par un alkyle en $C_1$-$C_4$ ou un alkylcarbonyle ou un benzoylamino éventuellement substitués.

6. Procédé selon les revendications 1 à 3, caractérisé en ce que l'on utilise des colorants en dispersion rongeables répondant à la formule I, dans laquelle K est le radical d'une 1- ou 2-naphtylamine répondant à la formule

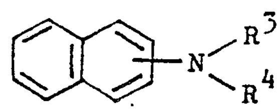

dans laquelle $R^3$ désigne l'hydrogène, un alkyle un alcényle et $R^4$ un alkyle, un alcényle, un cycloalkyle, un phényle, un alkylcarbonyle, un benzoyle, un alkylsulfonyle ou un phénylsulfonyle, dont les radicaux N-alkyle ou N-phényle peuvent le cas échéant être substitués et dont le noyau peut éventuellement être substitué par un alkyle en $C_1$-$C_4$, un alcoxy en $C_1$-$C_4$, le chlore, le brome, un aminosulfonyle, un aminosulfonyle N-N-mono- ou N,N-di-alkyl-substitué, les radicaux alkyle pouvant de leur côté être mono- ou poly-substitués le cas échéant, indépendamment les uns des autres, ou d'un 1- ou 2-naphtol dont le noyau peut le cas échéant être substitué par un aminocarbonyle, un mono- ou dialkyl- ou phénylamino-carbonyle, éventuellement substitués indépendamment les uns des autres, un alcoxy-, un alcénoxy-, un cycloalcoxy- ou un phénoxycarbonyle éventuellement substitués, un amino, un alkylcarbonylamino, un acénylcarbonylamino éventuellement substitués, un benzoylamino éventuellement substitué, un alkylsulfonylamino éventuellement substitué, un phénylsulfonylamino éventuellement substitué, un alkyle en $C_1$-$C_4$, un alcoxy en $C_1$-$C_4$, le chlore ou le brome, un aminosulfonyle, un mono- et di-alkylaminosulfonyle dont les radicaux alkyle peuvent le cas échéant être substitués indépendamment les uns des autres.

7. Procédé selon les revendications 1 à 3, caractérisé en ce que l'on utilise des colorants en dispersion rongeables répondant à la formule I, dans laquelle K est le radical d'une 6-hydroxypyridone-2-répondant à la formule II

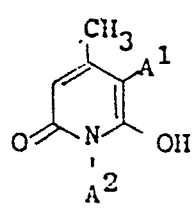

(II)

dans laquelle $A^1$ désigne un cyano, un nitro, un aminocarbonyle éventuellement substitué, l'hydrogène et $A^2$ l'hydrogène, un amino éventuellement substitué ou un alkyle en $C_1$-$C_8$ éventuellement substitué, ou d'un dérivé de l'aminopyridine répondant à la formule III

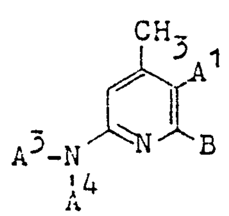

(III)

dans laquelle $A^3$ et $A^4$ désignent l'hydrogène ou un alkyle en $C_1$-$C_8$ éventuellement substitués

indépendamment l'un de l'autre, un alcényle en $C_3$-$C_5$, un cycloalkyle en $C_5$ ou $C_6$ et B est $NA^5A^6$ ou un alcoxy éventuellement substitué ou un alkylthio en $C_1$-$C_8$, et $A^5$ et $A^6$ désignent indépendamment l'un de l'autre un hydrogène ou un alkyle en $C_1$-$C_8$, éventuellement substitué, un alcényle en $C_3$-$C_5$, un cycloalkyle en $C_5$ ou $C_6$, et $A^1$ a les significations indiquées ci-dessus, ou d'un dérivé de l'aminopyridine répondant à la formule IV

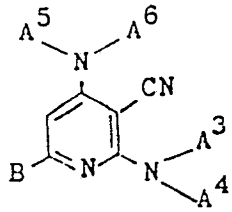

(IV)

dans laquelle $A^3$ à $A^6$ et B ont les significations indiquées ci-dessus.

8. Procédé selon les revendications 1 à 7, caractérisé en ce que l'on utilise des colorants en dispersion rongeables répondant à la formule I, dans laquelle les radicaux alkyle contenus dans $R^1$, $R^2$ et $A^3$ à $A^6$ des constituants de copulation contiennent 1 à 8 atomes de carbone, les radicaux alcényle 3 à 5 atomes de carbone, les radicaux cycloalkyle 5 ou 6 atomes de carbone et les radicaux aryle sont des radicaux phényle ou naphtyle.

9. Procédé selon les revendications 1 à 6, caractérisé en ce que l'on utilise des colorants en dispersion rongeables répondant à la formule I, dans laquelle les radicaux alkyle des constituants de copulation peuvent être non substitués ou sont substitués par les substituants hydroxy ; alcoxy en $C_1$-$C_4$ ; alcényloxy en $C_3$-$C_5$ ; phénoxy éventuellement substitué ; cycloalcoxy en $C_5$ ou $C_6$ ; hydroxyalcoxy, alkylcarbonyloxyalcoxy ou phénoxyalcoxy ayant 2 à 4 atomes de carbone dans le radical alcoxy ou alkylcarbonyle ; alcoxyalcoxy en $C_3$-$C_8$ ; hydroxyalcoxyalcoxy, alkylcarbonyloxyalcoxyalcoxy ou phényloxyalcoxyalcoxy ayant 4 à 12 atomes de carbone dans le radical alcoxyalcoxy et 2 à 4 atomes de carbone dans le radical alkylcarbonyle ; alcoxyalcoxyalcoxy en $C_5$-$C_{16}$ ; alkylcarbonyloxy en $C_2$-$C_4$ éventuellement substitué par un hydroxy, le chlore, le brome, un alcoxy en $C_1$-$C_4$ ou un phénoxy ; benzoyloxy éventuellement substitué ; alcoxycarbonyloxy ayant 1 à 4 atomes de carbone dans le groupe alcoxy, éventuellement substitué par un hydroxy, un chlore, un brome, un alcoxy en $C_1$-$C_4$ ou un phénoxy ; alcénoxycarbonyloxy ayant 3 à 5 atomes de carbone dans le groupe alcénoxy ; cycloalcoxycarbonyloxy ayant 5 ou 6 atomes de carbone dans le groupe cycloalcoxy ; phénoxycarbonyloxy éventuellement substitué ; monoalkylaminocarbonyloxy ayant 1 à 8 atomes de carbone dans le groupe alkylamino éventuellement substitué indépendamment les uns des autres par un hydroxy, un chlore, un brome, un alcoxy en $C_1$-$C_4$ ou un phénoxy ; monoarylaminocarbonyloxy éventuellement substitué ; phényle éventuellement substitué ; le chlore ; le brome ; un cyano ; et, si R est un alkyle éventuellement substitué, alcoxycarbonyle éventuellement substitué dans le groupe alcoxy, ayant 1 à 8 atomes de carbone ; alcényloxycarbonyle ayant 3 à 5 atomes de carbone dans le groupe alcénoxy ; cycloalcoxycarbonyle ayant 5 ou 6 atomes de carbone dans le groupe cycloalcoxy ; phénoxycarbonyle ayant un radical phénoxy éventuellement substitué, et les radicaux naphtyle ou phényle peuvent être mono- ou di-substitués par le fluor, le chlore, le brome, un trifluorométhyle, un alkyle en $C_1$-$C_4$, un alcoxy en $C_1$-$C_4$.

10. Matière textile colorée par le procédé selon les revendications 1 à 9.